# EUROPEAN PATENT APPLICATION

(11) **EP 4 032 910 A1**
(43) Date of publication of application: **27.07.2022**
(21) Application number: 21152969.8
(22) Date of filing: 22.01.2021
(51) Int. Cl.: C07K 16/28, C07K 16/30, C07K 16/44, A61P 35/00

(54) **BISPECIFIC BINDING AGENT THAT BINDS TO CD3 AND A FLUOROPHORE**

(71) Applicant: ETH Zurich, 8092 Zurich (CH); Universität Zürich, 8006 Zürich (CH)
(72) Inventor: NERI, Dario, 8853 Lachen (CH); MYBURGH, Renier, 5000 Aarau (CH); MANZ, Markus, 8032 Zürich (CH); VOLTA, Laura, 8052 Zürich (CH)

(57) **Abstract**

The present invention relates to a bispecific binding agent comprising at least a first binding domain and a second binding domain, wherein said first binding domain binds to an organic fluorophore and wherein said second binding domain binds to CD3, and to a combination thereof with a labelled binding agent that binds specifically to a target antigen, wherein the labelled binding agent is labelled with an organic fluorophore

## Description

### Field of the invention

The present invention relates to a bispecific binding agents.

### Background

Bispecific, T-cell engaging and activating antibodies and chimeric antigen receptor (CAR) T-cells (CAR T) cells are promising antibody-based approaches to treat various types of cancers (or other diseases as autoimmunity, inflammation or infection). Both therapies rely on the (re)direction of the killing activity of T cells towards target cells.

Bispecific antibodies are biopharmaceuticals, able to simultaneously bind two different antigens with the intention to link an effector cell (e.g. T-cell) to a target-antigen expressing cell, leading to effector-cell mediated killing of the target-antigen carrying cell. Specifically, bispecific antibodies can bind CD3 on T-cells and a tumor-associated antigen, bridging together tumor cells and effector T-cells. The cross-linking leads to T-cell activation and elicits a potent and selective (re)targeting of their cytolytic activity to the target-carrying cell in an MHC-independent manner. Clinical-stage bispecific antibodies range from small proteins, consisting of two linked antigen-binding fragments, to large immunoglobulin G (IgG)-like molecules with additional domains attached.

CAR-T cells are genetically modified T-cells that overexpress an artificial CAR which usually is comprised of a single-chain variable fragment (scFv) binding domain linked to the T-cell via a flexible hinge and a transmembrane domain. Additional CAR components, the intracellular co-stimulatory and stimulatory domains, mediate the activation signal. The scFv on the surface re-directs CAR-T cell cytotoxic activity in an MHC-independent manner against the target cells where the cognate antigen is expressed.

One bispecific T-cell engager (BiTE^{™}), consisting of two scFvs against CD3 and CD19 has gained regulatory approval (Blinatumomab; Blycinto^{®}) as well as two CAR-T cells products (Kymriah^{®} and Yescarta^{®}) as salvage therapies for certain subtypes of B-cell leukemia or lymphoma.

However, despite high rates of initial response, relapse is observed in a notable proportion of patients. Bispecific antibodies and CAR-T cells, capable of targeting a single tumor cell antigen, are unable to address antigen escape or overcome tumor heterogeneity. Outcome improvement by use of combinatorial strategies would require the development of a new GMP product for each new antigen one may want to target.

Compared to the development of a recombinant protein, GMP production of CAR-T cells accounts for the costs of a personalized medicine, where each batch is manufactured from patients' cells, thus limiting its clinical applicability. Moreover, while the activity of the antibodies can be controlled by injection schedule, this on-off safety switch is currently not possible when using CAR-T cells. Their activation lacks downstream control mechanisms after injection which may result in severe side effects derived from systemic toxicities or on-target, off-tumor effects due to the strong level of CAR T-cell activation.

However, so far, only a small selection of bispecifc T cell engaging binding agents is available on the market. This severely restricts the clinical potential of T cell engaging therapy to diseases that exhibit targets for which one of the few bispecifc T cell engaging binding agents is already available, and, in case the respective disease develops a resistance against said therapy, leaves the practitioner with no suitable 2^{nd} line therapy.

### Summary of the Invention

The present invention provides a bispecific binding agent comprising at least a first binding domain and a second binding domain, wherein said first binding domain binds to CD117/c-KIT and wherein said second binding domain binds to CD3. The invention and general advantages of its features and embodiments will be discussed in detail below.

Such new constructs are called "Universal T cell engaging and activating bispecific antibodies", abbreviated UniTEA herein.

### Brief Description of the Figures

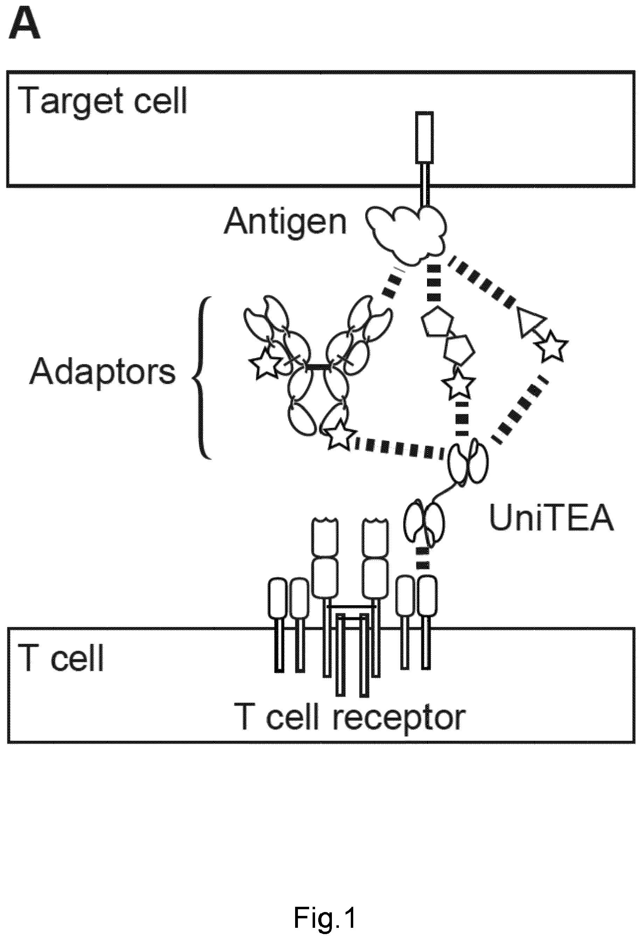

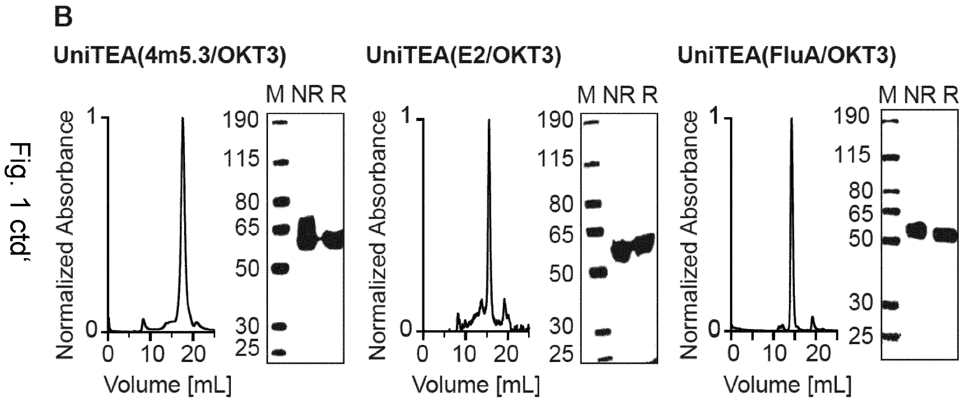

Figure 1. Schematic representation and characterization of UniTEAs.
   (A) The Universal T-cell engaging and activating bispecific binding agent (UniTEA) has dual specificity for human CD3 (e.g. CD3ε) expressed on T-cells and for fluorescein. T-cell cytotoxic activity is only elicited in presence of a fluorophore-tagged, target-bound adaptor. The fluorophore is shown as a star ( 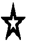 ). Different types of binders are shown that can be used as adaptors, namely an antibody (left part, symbolized by the IgG-like shape, yet also encompassing other fragments or derivatives thereof, like e.g. a scFv or diabody, as well as a small molecule (middle part) or a peptide (right part)),
   (B) Size exclusion chromatography profiles and SDS PAGE gels showing the purity and molecular weight of three different UniTEA formats, called 4m5.3/BlinCD3, E2/BlinCD3 and FluA/BlinCD3.

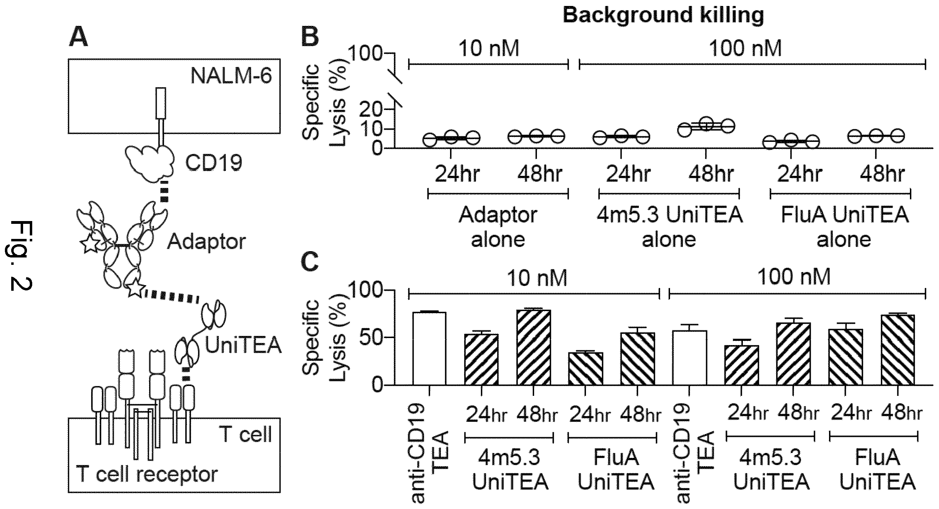

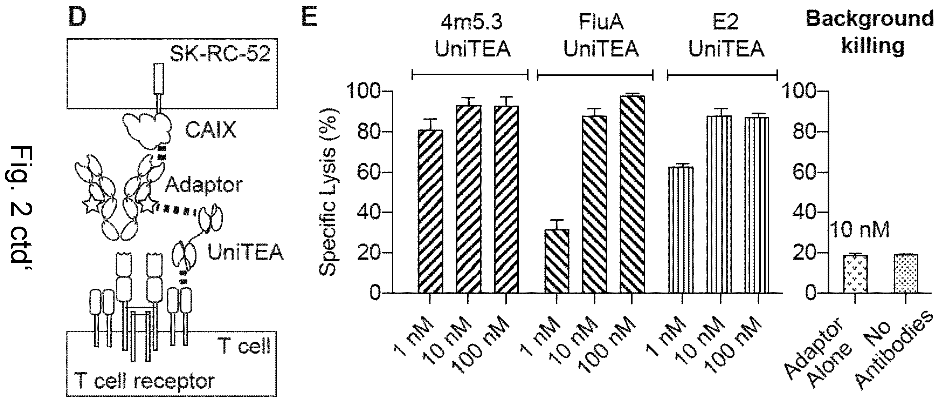

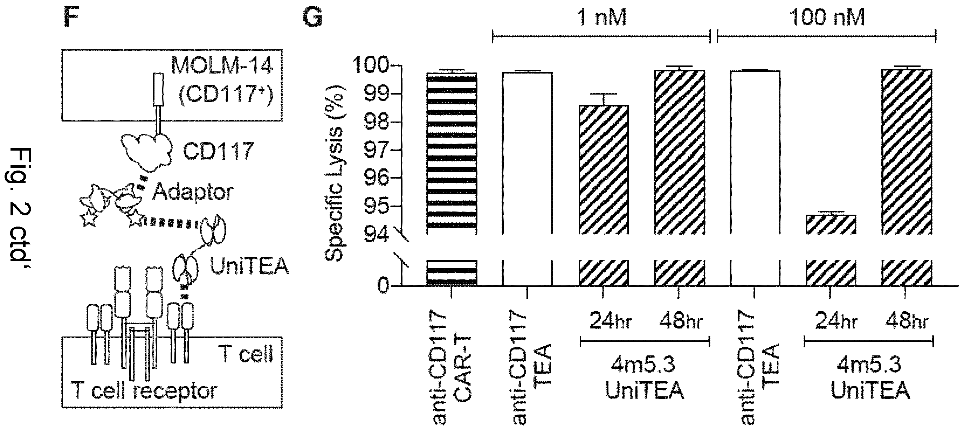

Figure 2. T cell-mediated depletion of CD19, CAIX and CD117-expressing cell lines *in vitro.*
   (A) NALM-6 (CD19⁺) B-ALL cell line was used to compare the T cell mediated killing elicited by the 4m5.3/BlinCD3 and FluA/BlinCD3 UniTEA formats to Blinatumomab^{™} (anti-CD19/BlinCD3 T-cell Engaging and Activating bispecific binding agent, TEA). Target cells and T-cells were co-incubated for 24hrs and 48hrs at 10:1 E:T ratio.
   (B) Background killing of only UniTEA or commercial anti human CD19 IgG-FITC adaptor molecule at the indicated concentrations.
   (C) Equimolar concentration of 4m5.3/BlinCD3 and FluA/BlinCD3 UniTEAs elicited similar T-cell mediated target cell lysis as Blinatumomab^{™} when 10 nM of adaptor were added.
   (D) Renal cell carcinoma cell line SK-RC-52 (CAIX⁺) was used to assess the T cell mediated killing elicited by 4m5.3/BlinCD3, FluA/BlinCD3 and E2/BlinCD3 UniTEAs in presence of 10nM site-specifically fluoresceinated anti-CAIX IgG.
   (E) All three UniTEA formats induced T-cell mediated tumor cell lysis in a concentration-dependent fashion after 24hrs at 10:1 E:T ratio. Background killing after co-culturing T- and tumor cells alone or by adding only 10nM adaptor molecule is also reported.
   (F) A comparison between CAR T cell, TEA and UNiTEA was performed by targeting CD117 on MOLM14 (CD117⁺) AML cells. The 4m5.3/BlinCD3 UNiTEA (BiTE format) was used in combination with a site-specifically fluoresceinated anti-CD 117 diabody (Db). Target cells and effector cells were co-incubated for 24hrs and 48hrs at 1:1 E:T ratio.
   (G) Addition of 4m5.3/BlinCD3 UniTEA in presence of 10nM fluorescinated anti-CD117 Db resulted in similar T-cell mediated target cell lysis at 24 and 48 hours as direct anti-CD117 CAR-T cells and anti-CD117/BlinCD TEA.

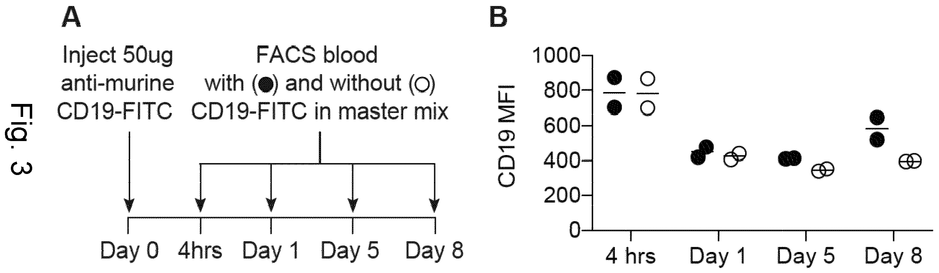

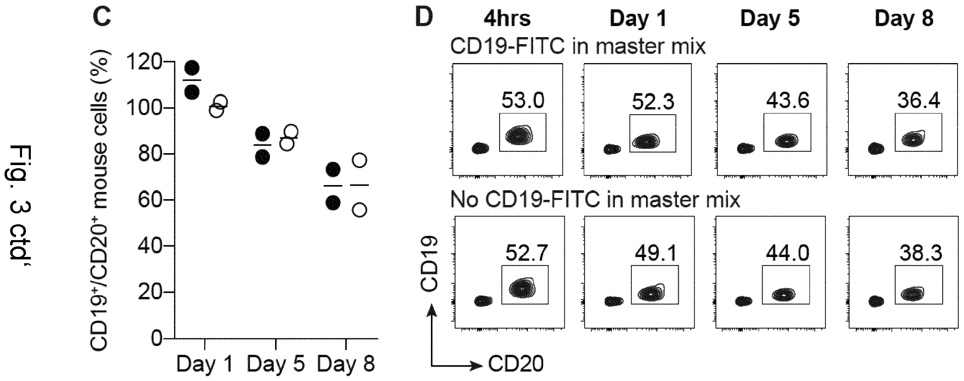

Figure 3. *In vivo* persistence of FITC-antibodies.
   (A) Commercially available anti murine CD19 (mCD19) IgG-FITC was injected i.v. in two C57BL/6J mice (50 ug/mouse). Peripheral blood was analyzed by FACS at different time points over 8 days.
   (B) Mean fluorescence intensity (MFI) of mCD19⁺ cells stained at each time point with and without additional mCD19 IgG-FITC, represented by filled and hollow circles, respectively.
   (C) Live mCD19⁺/mCD20⁺ B cells, expressed as percentage of mCD19⁺/mCD20⁺ cells at each time point compared to the baseline number after 4hrs, stained with and without additional mCD19 IgG-FITC, represented by filled and hollow circles, respectively.
   (D) Representative FACS plots showing the peripheral blood CD19⁺CD20⁺ murine B cell population at indicated time points with and without additional CD19-FITC labelling.

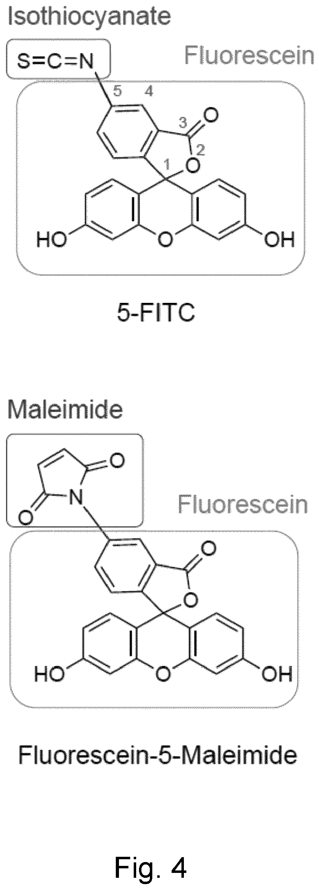

Figure 4: Structures of FITC and Fluorescein-5-Maleimide (the latter is used for site specific conjugation of FITC to an antibody that is labelled with a terminal Cystein residue)
Figure 5: Overview of different bispecific antibody formats. This figure shows the different formats of bispecific antibodies that can be used in the present invention. It has been taken from Spiess et al. 2015, the content of which is incorporated herein by reference, in particular to account for a potential loss of color information..

### Detailed Description of the Invention

Before the invention is described in detail, it is to be understood that this invention is not limited to the particular component parts of the devices described or process steps of the methods described as such devices and methods may vary. It is also to be understood that the terminology used herein is for purposes of describing particular embodiments only, and is not intended to be limiting. It must be noted that, as used in the specification and the appended claims, the singular forms "a", "an", and "the" include singular and/or plural referents unless the context clearly dictates otherwise. It is moreover to be understood that, in case parameter ranges are given which are delimited by numeric values, the ranges are deemed to include these limitation values.

It is further to be understood that embodiments disclosed herein are not meant to be understood as individual embodiments which would not relate to one another. Features discussed with one embodiment are meant to be disclosed also in connection with other embodiments shown herein. If, in one case, a specific feature is not disclosed with one embodiment, but with another, the skilled person would understand that does not necessarily mean that said feature is not meant to be disclosed with said other embodiment. The skilled person would understand that it is the gist of this application to disclose said feature also for the other embodiment, but that just for purposes of clarity and to keep the specification in a manageable volume this has not been done.

Furthermore, the content of the prior art documents referred to herein is incorporated by reference. This refers, particularly, for prior art documents that disclose standard or routine methods. In that case, the incorporation by reference has mainly the purpose to provide sufficient enabling disclosure, and avoid lengthy repetitions.

According to a first aspect of the invention, a bispecific binding agent comprising at least a first binding domain and a second binding domain, wherein said first binding domain binds to an organic fluorophore and wherein said second binding domain binds to CD3.

Such new constructs are called "Universal T cell engaging and activating bispecific antibodies", abbreviated UniTEA herein.

As used herein, the term "organic fluorophore" relates to organic small molecules that have the capacity to re-emit light upon light excitation. Organic fluorophores, as disclosed herein, have a maximum molecular that does not exceed 2500 Daltons. The term "organic fluorophore" excludes fluorescent proteins like green fluorescent protein (GFP), which has a molecular weight of 27 kDa and several phycobiliproteins, which have molecular weights of about 240kDa.

In some embodiments, the organic fluorophore belongs to at least one of the groups selected from the list consisting of
- Xanthene derivatives, like e.g. fluorescein, rhodamine, Oregon green, eosin, and Texas red
- Cyanine derivatives, like e.g. cyanine, indocarbocyanine, oxacarbocyanine, thiacarbocyanine, and merocyanine
- Squaraine derivatives and ring-substituted squaraines, including Seta and Square dyes
- Squaraine rotaxane derivatives, like e.g. See Tau dyes
- Naphthalene derivatives (dansyl and prodan derivatives)
- Coumarin derivatives
- Oxadiazole derivatives, like e.g. pyridyloxazole, nitrobenzoxadiazole and benzoxadiazole
- Anthracene derivatives, like e.g. anthraquinones, including DRAQ5, DRAQ7 and CyTRAK Orange
- Pyrene derivatives, like e.g. cascade blue, etc.
- Oxazine derivatives, like e.g. Nile red, Nile blue, cresyl violet, oxazine 170, etc.
- Acridine derivatives, like e.g. proflavin, acridine orange, acridine yellow, etc.
- Arylmethine derivatives, like e.g. auramine, crystal violet, malachite green
- Tetrapyrrole derivatives, like e.g. porphin, phthalocyanine, bilirubin, and/or
- Dipyrromethene derivatives, like e.g. BODIPY, aza-BODIPY

The inventors have found, surprisingly, that such constructs can be used, together with a second binding agent that is labelled with a corresponding fluorophore (also called "adaptor" herein), to bring T cell engagement therapy to almost every conceivable target in a patient's body.

Hence, a modular system is provided which facilitates the application of T cell engagement therapy to targets that have so far not been available for this approach. Further, such system may facilitate and accelerate regulatory approval for T cell engagement therapies against new targets.

The resulting UniTEA and fluorophore-tagged adaptor complex facilitates formation of a cytolytic synapse between the CD3 expressing T-cells and target cells, leading to effective target cell killing.

The UniTEA can be provided as an off-the-shelf GMP-manufactured biopharmaceutical and can be then used in combination with clinically approved therapeutic antibodies or other target-binders (called "adaptors" herein), which will be minimally modified (fluorophore-tagged) in order to make them suitable adaptors to redirect T-cell cytotoxicity to the respective target of interest.

Since organic fluorophores can be linked to a wide range of adaptors, T-cells can be redirected universally to any target cell of choice, provided there is an available clinical-grade bridge. Moreover, the antibodies (or peptides, small molecules, or any other antigen-binder) that would serve as appropriate adaptors will have their functionality enhanced due to UniTEs recruiting T cells and redirecting them via the adaptors.

The UniTEA modular system would allow an on-demand T-cell activation, which would be elicited only when the UniTEA is present concomitantly to the fluorophore-tagged, target bound adaptor, while no T-cell cytotoxic activity can be elicited in the absence of the UniTEA even when fluorophore-tagged bridges may persist in the body. Therefore, UniTEAs short half-life (∼30 min) exerts a precise control over endogenous T cell activity allowing for a fine tuning of the effects by dosing.

Antigen negative relapse or tumor heterogeneity would be addressed by exploiting combinatorial strategies that can be further personalized for each patient or disease, respectively. This system therefore allows existing clinically used target-binding molecules to be utilized in combinations or serially, allowing to target a wider and more heterogeneous range of tumor cell antigens (hematologic and non-hematologic tumors) overcoming the limitations of mono-specific tumor antigen targeting approaches.

Compared to the manufacturing costs of novel single- or multi-valent CAR-T cells as well as modular CAR-T cells which all require a dedicated GMP facility to genetically engineer patients' T-cells, the investment for developing a protein is sensibly lower. The advantages of generating a single universal bispecific protein supersede the production of *de novo* bispecific antibodies. The development of UniTE molecules would represent an alternative to the very demanding multivalent antibodies and CAR T-cells, while keeping the personalized advantages of a tailored therapeutical and the GMP production cost of a single recombinant protein.

According to one embodiment, said second binding domain binds to the ε chain of CD3 and/or the γ chain of CD3.

The T cell receptor (TCR) complex is composed of ligand-binding subunits (TCRα and TCRβ) and signal transducing subunits (CD3γ,CD3δ,CD3ε and CD3ζ [CD247]) The ligand of the TCR consists of a peptide antigen bound to major histocompatibility complex (MHC) class I or class II molecules. Assembly studies, transfection and reconstitution experiments, and detergent dissociation studies suggest that the TCR complex components are organized as dimers [reviewed in. CD3ε forms non-covalently-bound alternate dimers with CD3γ and CD3δ, TCRα forms disulfide-linked dimers with TCRβ, and CD3ζ is expressed in the form of disulfide-linked homodimers.

T cells can be engaged by means of binding molecules capable of binding to CD3, in particular to CD3ε, the invariant signaling component of the T cell receptor complex. This takes advantage of the ability to harness polyclonal populations of cytotoxic T cells [CD8+, CD4+, including regulatory T cells (Tregs)] and is not dependent on MHC class I for antigen presentation. The monovalent binding of CD3 does not activate the TCR unless target cell binding is engaged, as will be discussed below, in a combination of the bispecific binding agent and the labelled binding agent. In such way, engagement of T cells drives activation and resultant proliferation of the T cells which contributes to enhanced efficacy. Moreover. T cell engagement contributes to serial lysis which further enhances efficacy.

According to one embodiment of the invention, the bispecific binding agent is in the format of a full-length antibody or an antibody fragment, the latter retaining target binding properties.

Generally, a large number of formats for bispecifc antibody has been evaluated. For a review, see, e.g., Spiess et al. (2015), the content of which is incorporated herein by reference. Bispecific antibodies are classified into five distinct structural groups:
(i) bispecific IgG (BsIgG)
(ii) IgG appended with an additional antigen-binding moiety
(iii) bispecific antibody fragments
(iv) bispecific fusion proteins and
(v) bispecific antibody conjugates

Further, it is often differentiated between
(i) asymmetric bispecific antibodies (heteropolymeric antibodies with different chains comprising binding moieties to different antigens) and
(ii) symmetric bispecific antibodies, (heteropolymeric or monomeric/single chain antibodies, where the different binding moieties to different antigens are on the same chain)

Generally, small formats, which are often in the monomeric/single chain configuration) have a short serum half life, which makes frequent or even constant dosing necessary, yet provides the option to interrupt treatment immediately upon occurrence of side effects, like cytokine storms.

Asymmetric bispecific antibodies often pose problems when it comes to post-translational pairing of the different chains In the simultaneous expression of four different antibody chains (heavy and light chain for each specificity) irregular pairing can occur, leading to 10 different products, out of which only one is the correct variant. These mispairing issues can however be tackled with different technological approaches (see Wang et al. 2018).

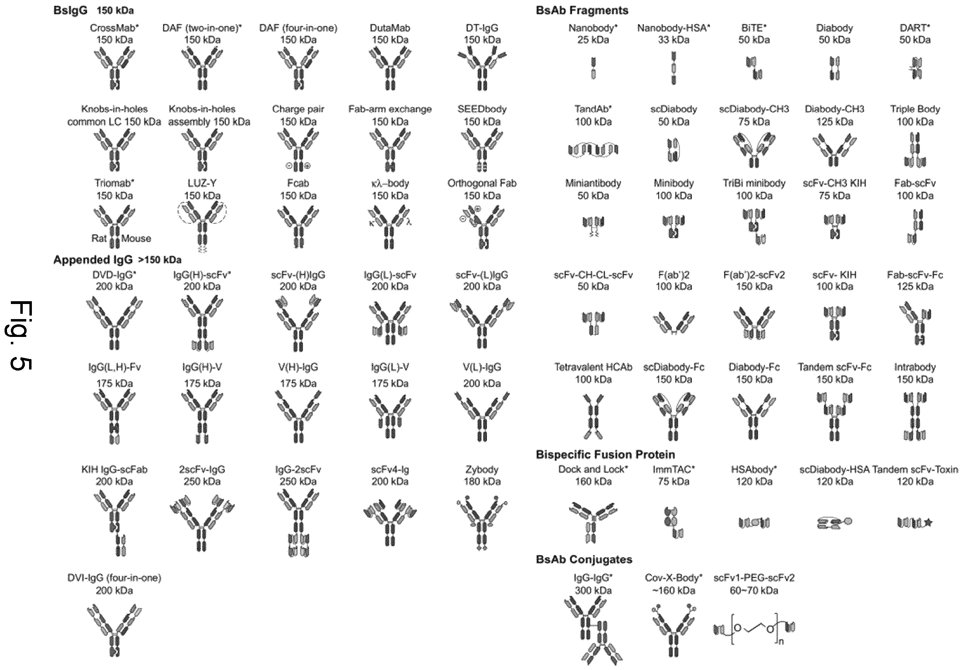

See Fig. 5 for an overview of the different formats. Each of these formats brings different properties and advantages and can hence be chosen as a preferred format, *inter alia,*
- binding valency for each antigen,
- geometry of antigen-binding sites, c
- correctness of chain pairing,
- pharmacokinetic half-life
- and in some cases effector functions.

According to embodiments of the invention, the bispecific binding agent is in the format of at least one selected from the group consisting of
- DVD-Ig
- Knobs in Holes
- Anticalin-scFv
- Crossmab
- BiTE^{™}
- Nanobody
- Diabody
- DART^{™}
- TandAb^{™,} and/or
- scDb-scFv

These formats are well known to the skilled artisan (see Spiess et al. (2015) for the definitions). The skilled artisan can use the variable domains and/or sets of CDRs as disclosed herein to create the above mentioned formats by routine measures.

According to one embodiment of the invention, the bispecific binding agent is in the format of a BiTE.

The BiTE format essentially consists of two scFv formatted antibodies joined to one another by means of a peptide linker, usually a linker comprising the amino acid sequence GGGGs ("G4S"). The resulting single chain has a weight of about 55 kD. Said BiTE can have the following formats (with the dash between the variable domains representing optional linkers):
- VH₁-VL₁-VL₂-VH₂
- VL₁-VH₁-VH₂-VL₂
- VL₁-VH₁-VL₂-VH₂
- VH₁-VL₁-VH₂-VL₂

Because of its small size, the BiTE format is associated with a very rapid clearance profile in patients. Blinatumomab, the only clinically-approved BiTE on the market, is administered as 28-day continuous infusion, thanks to the use of an intravenous pump with constant flow. In such way, the opportunity exists to immediately suspend antibody administration if serious adverse events occur.

The scDb-scFv is similar to the BiTE format, only with the difference that the variable domains for the first or second target are duplicated, es e.g. shown in the following:
- VH₁-VL₁-VH₁-VL₁-VL₂-VH₂
- VL₁-VH₁-VL₁-VH₁-VH₂-VL₂
- VL₁-VH₁-VL₁-VH₁-VL₂-VH₂
- VH₁-VL₁-VH₁-VL₁-VH₂-VL₂

The TandAb (Tandem Diabody) format is for example as follows:

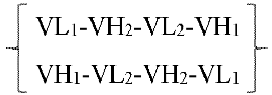

The DART format is a dimeric format where two chains are connected to one another by a cysteine linker:
- Chain 1: VL₁₋VH₂
- Chain 2: VH₁₋VL₂

Anticalin proteins are artificial proteins that are able to bind to antigens, either to proteins or to small molecules. They are not structurally related to antibodies, which makes them a type of antibody mimetic. Instead, they are derived from human lipocalins which are a family of naturally binding proteins. Anticalin proteins are being used in lieu of monoclonal antibodies, but are about eight times smaller with a size of about 180 amino acids and a mass of about 20 kDa.

According to one embodiment, said first binding domain of the bispecific binding agent binds to Fluorescein, preferably Fluorescein when bound to a protein (conjugated Fluorescein).

As used herein, the terms "Fluorescein", "Fluorescein isothiocyanate" and "FITC" are being used interchangeably. Binding agents, including antibodies binding to Fluorescein, preferably Fluorescein when bound to a protein (conjugated Fluorescein), are widely available. While some embodiments will be discussed in the following, reference is also made to the internet resource Biocomepare, which lists >78000 Anti-FITC Antibody Products, with ab19224 (Abcam, Goat polyclonal IgG) and LS C34608 (LSBio, Monoclonal Mouse FITC Antibody (clone F4/1, IHC), to name only a few.

According to one embodiment, said first binding domain of the bispecific binding agent which binds to Fluorescein or conjugated Fluorescein comprises the anticalin FluA (SEQ ID NO: 47), which is disclosed in Vopel et al. (2005), the content of which is incorporated herein by reference.

As discussed elsewhere herein, anticalin proteins are artificial proteins that are able to bind to antigens, either to proteins or to small molecules. They are derived from human lipocalins which are a family of naturally binding proteins.

Preferably, said first binding domain of the bispecific binding agent which binds to Fluorescein or conjugated Fluorescein comprises an anticalin FluA that has a sequence identity of ≥ 80%, ≥ 81%, ≥ 82%, ≥ 83%, ≥ 84%, ≥ 85%, ≥ 86%, ≥ 87%, ≥ 88%, ≥ 89%, ≥ 90%, ≥ 91%, ≥ 92%, ≥ 93%, ≥ 94%, ≥ 95%, ≥ 96%, ≥ 97%, ≥ 98%, and most preferably ≥ 99 % to SEQ ID NO: 47.

In the following, embodiments will be described in which specific antibody sequences are referred to. In some embodiments, alternative CDR (complementarity determining regions) sequences will be disclosed. These embodiments relate to the same antibody, but only to different nomenclatures according to which the CDRs were defined.

As used herein, the term "CDR" or "complementarity determining region" is intended to mean the non-contiguous antigen combining sites found within the variable region of both heavy and light chain polypeptides. These particular regions have been described by Kabat et al. (1977), Kabat et al. (1991), Chothia et al. (1987) and MacCallum et al., (1996) where the definitions include overlapping or subsets of amino acid residues when compared against each other. Nevertheless, application of either definition to refer to a CDR of an antibody or grafted antibodies or variants thereof is intended to be within the scope of the term as defined and used herein. The amino acid residues which encompass the CDRs as defined by each of the above cited references are set forth below in Table 1 as a comparison. Note that this numbering may differ from the CDRs that acre actually disclosed in the enclosed sequence listing, because CDR definitions vary from case to case.

**Table 1: CDR definitions**

| | **Kabat** | **Chothia** | **MacCallum** |
|---|---|---|---|
| VH CDR1 | 31-35 | 26-32 | 30-35 |
| VH CDR2 | 50-65 | 53-55 | 47-58 |
| VH CDR3 | 95-102 | 96-101 | 93-101 |
| VL CDR1 | 24-34 | 26-32 | 30-36 |
| VL CDR2 | 50-56 | 50-52 | 46-55 |
| VL CDR3 | 89-97 | 91-96 | 89-96 |

As used herein, the term "framework" when used in reference to an antibody variable region is entered to mean all amino acid residues outside the CDR regions within the variable region of an antibody. Therefore, a variable region framework is between about 100-120 amino acids in length but is intended to reference only those amino acids outside of the CDRs.

In one embodiment, the term "capable to bind to target X" is to be understood as meaning binding with a sufficient binding affinity. In such embodiment, the respective binding domain binds the target with a K_{D} of 10⁻⁴ or smaller. K_{D} is the equilibrium dissociation constant, a ratio of k_{off}/kₒₙ, between the antibody and its antigen. K_{D} and affinity are inversely related. The K_{D} value relates to the concentration of antibody (the amount of antibody needed for a particular experiment) and so the lower the K_{D} value (lower concentration) and thus the higher the affinity of the binding domain. The following table shows typical K_{D} ranges of monoclonal antibodies:

**Table 2. K_{D} and Molar Values**

| **K_{D} value** | **Molar range** |
|---|---|
| 10⁻⁴ to 10⁻⁶ | Micromolar (µM) |
| 10⁻⁷ to 10⁻⁹ | Nanomolar (nM) |
| 10⁻¹⁰ to 10⁻¹² | Picomolar (pM) |
| 10⁻¹³ to 10⁻¹⁵ | Femtomolar (fM) |

Acording to one embodiment, the first binding domain which binds to Fluorescein or conjugated Fluorescein
a) comprises a set of heavy chain/light chain complementarity determining regions (CDR) comprised in the heavy chain/light variable region sequence pair of one of the anti Fluorescein antibodies E2 or 4m5.3, as set forth in SEQ ID NOs 27 and 31, or SEQ ID NOs 36 and 40, respectively
b) comprises the set of heavy chain/light chain complementarity determining regions (CDR) comprising the following sequences
   - HC CDR1 (SEQ ID NO 28)
   - HC CDR2 (SEQ ID NO 29)
   - HC CDR3 (SEQ ID NO 30)
   - LC CDR1 (SEQ ID NO 32)
   - LC CDR2 (SEQ ID NO 33), and
   - LC CDR3 (SEQ ID NO 34)
c) comprises the set of heavy chain/light chain complementarity determining regions (CDR) comprising the following sequences
   - HC CDR1 (SEQ ID NO 37)
   - HC CDR2 (SEQ ID NO 38)
   - HC CDR3 (SEQ ID NO 39)
   - LC CDR1 (SEQ ID NO 41)
   - LC CDR2 (SEQ ID NO 42), and
   - LC CDR3 (SEQ ID NO 43)
d) comprises the heavy chain/light chain complementarity determining regions (CDR) of b) or c), with the proviso that at least one of the CDRs has up to 3 amino acid substitutions relative to the respective SEQ ID NO 28 - 30, 32 - 34, 37 - 39 or 41 - 43, and/or
e) comprises the heavy chain/light chain complementarity determining regions (CDR) of b), with the proviso that at least one of the CDRs has a sequence identity of ≥ 66 % to the respective SEQ ID NO 28 - 30, 32 - 34, 37 - 39 or 41 - 43,
wherein the CDRs are embedded in a suitable protein framework so as to be capable to bind to Fluorescein or conjugated Fluorescein.

The anti-fluorescein antibody E2 is disclosed in Vaughan et al. (1996), the content of which is incorporated herein by reference. The anti-fluorescein antibody 4m5.3 is disclosed in Boder et al. (2000), the content of which is incorporated herein by reference.

Preferably, and this applies to all CDR sets disclosed herein, at least one CDR has up to 2 amino acid substitutions, and more preferably up to 1 amino acid substitutions

Preferably, and this applies to all CDR sets disclosed herein, at least one of the CDRs has a sequence identity of ≥ 67%, ≥ 68%, ≥ 69%, ≥ 70%, ≥ 71%, ≥ 72%, ≥ 73%, ≥ 74%, ≥ 75%, ≥ 76%, ≥ 77%, ≥ 78%, ≥ 79%, ≥ 80%, ≥ 81%, ≥ 82%, ≥ 83%, ≥ 84%, ≥ 85%, ≥ 86%, ≥ 87%, ≥ 88%, ≥ 89%, ≥ 90%, ≥ 91%, ≥ 92%, ≥ 93%, ≥ 94%, ≥ 95%, ≥ 96%, ≥ 97%, ≥ 98%, and most preferably ≥ 99 % to the respective SEQ ID NO.

As used herein, the term "% sequence identity", has to be understood as follows: Two sequences to be compared are aligned to give a maximum correlation between the sequences. This may include inserting "gaps" in either one or both sequences, to enhance the degree of alignment. A % identity may then be determined over the whole length of each of the sequences being compared (so-called global alignment), that is particularly suitable for sequences of the same or similar length, or over shorter, defined lengths (so-called local alignment), that is more suitable for sequences of unequal length. In the above context, an amino acid sequence having a "sequence identity" of at least, for example, 95% to a query amino acid sequence, is intended to mean that the sequence of the subject amino acid sequence is identical to the query sequence except that the subject amino acid sequence may include up to five amino acid alterations per each 100 amino acids of the query amino acid sequence. In other words, to obtain an amino acid sequence having a sequence of at least 95% identity to a query amino acid sequence, up to 5% (5 of 100) of the amino acid residues in the subject sequence may be inserted or substituted with another amino acid or deleted. Methods for comparing the identity and homology of two or more sequences are well known in the art. The percentage to which two sequences are identical can for example be determined by using a mathematical algorithm. A preferred, but not limiting, example of a mathematical algorithm is integrated in the BLAST family of programs, e.g. BLAST or NBLAST program and FASTA. Sequences which are identical to other sequences to a certain extent can be identified by these programmes. Furthermore, programs available in the Wisconsin Sequence Analysis Package, version 9.1 for example the programs BESTFIT and GAP, may be used to determine the % identity between two polypeptide sequences. If herein reference is made to an amino acid sequence sharing a particular extent of sequence identity to a reference sequence, then said difference in sequence is preferably due to conservative amino acid substitutions. Preferably, such sequence retains the activity of the reference sequence, e.g. albeit maybe at a slower rate.

Preferably, and this applies to all CDR sets disclosed herein, at least one of the CDRs has been subject to CDR sequence modification, including
- affinity maturation
- reduction of immunogenicity

Affinity maturation in the process by which the affinity of a given antibody is increased *in vitro.* Like the natural counterpart, *in vitro* affinity maturation is based on the principles of mutation and selection. It has successfully been used to optimize antibodies, antibody fragments or other peptide molecules like antibody mimetics. Random mutations inside the CDRs are introduced using radiation, chemical mutagens or error-prone PCR. In addition, the genetic diversity can be increased by chain shuffling. Two or three rounds of mutation and selection using display methods like phage display usually results in antibody fragments with affinities in the low nanomolar range. For principles see Eylenstein et al. (2016), the content of which is incorporated herein by reference.

Humanized antibodies contain murine-sequence derived CDR regions that have been engrafted, along with any necessary framework back-mutations, into human sequence-derived V regions. Hence, the CDRs themselves can cause immunogenic reactions when the humanized antibody is administered to a patient. Methods of reducing immunogenicity caused by CDRs are disclosed in Harding et al. (2010), the content of which is incorporated herein by reference. According to one embodiment of the invention, the framework is a human VH/VL framework. VH stands for heavy chain variable domain of an IgG shaped antibody, while VL stands for light chain variable domain (kappa or lambda).

According to one embodiment of the invention, the first binding domain which binds to Fluorescein or conjugated Fluorescein comprises
a) the variable domains of one antibody selected from the group consisting of E2 or 4m5.3
b) the heavy chain/light chain variable domains (VD)
   - HC VD (SEQ ID NO 27 or 36), and
   - LC VD (SEQ ID NO 31 or 40)
c) the heavy chain/light chain variable domains (VD) of b), with the proviso that
   - the HCVD has a sequence identity of ≥ 80 % to the respective SEQ ID NO 27 or 36, and/or
   - the LCDVD has a sequence identity of ≥ 80 % to the respective SEQ ID NO 31 or 40
d) the heavy chain/light chain variable domains (VD) of b), with the proviso that at least one of the HCVD or LCVD has up to 10 amino acid substitutions relative to the respective SEQ ID NOs,
wherein said bispecific binding agent is still capable to bind to Fluorescein or conjugated Fluorescein.

A "variable domain" when used in reference to an antibody or a heavy or light chain thereof is intended to mean the portion of an antibody which confers antigen binding onto the molecule and which is not the constant region. The term is intended to include functional fragments thereof which maintain some of all of the binding function of the whole variable region. Variable region binding fragments include, for example, functional fragments such as Fab, F(ab)₂, Fv, single chain Fv (scfv) and the like. Such functional fragments are well known to those skilled in the art. Accordingly, the use of these terms in describing functional fragments of a heteromeric variable region is intended to correspond to the definitions well known to those skilled in the art. Such terms are described in, for example, Huston et al., (1993) or Plückthun and Skerra (1990).

Preferably, and this applies to all variable domains disclosed herein, at least one of the variable domains has ≤ 9, ≤ 8, ≤ 7, ≤ 6, ≤ 5, ≤ 4, ≤ 3, ≤ 2, ≤1 amino acid substitutions relative to the respective SEQ ID NO.

Preferably, and this applies to all variable domains disclosed herein, at least one of the variable domains has a sequence identity of ≥ 81 %, ≥ 82 %, ≥ 83 %, ≥ 84 %, ≥ 85 %, ≥ 86 %, ≥ 87 %, ≥ 88 %, ≥ 89%, ≥ 90 %, ≥ 91 %, ≥ 92%, ≥ 93 %, ≥ 94%, ≥ 95 %, ≥ 96 %, ≥ 97 %, ≥ 98 %, ≥ 99 to the respective SEQ ID NO.

Preferably, and this applies to all variable domains disclosed herein, at least one amino acid substitution in any of the variable domain disclosed herein is a conservative amino acid substitution

A "conservative amino acid substitution" has a smaller effect on antibody function than a non-conservative substitution. Although there are many ways to classify amino acids, they are often sorted into six main groups on the basis of their structure and the general chemical characteristics of their R groups.

In one embodiment, a "conservative amino acid substitution" is one in which the amino acid residue is replaced with an amino acid residue having a similar side chain. For example, families of amino acid residues having similar side chains have been defined in the art. These families include amino acids with
- basic side chains (e.g., lysine, arginine, histidine),
- acidic side chains (e.g., aspartic acid, glutamic acid),
- uncharged polar side chains (e.g., glycine, asparagine, glutamine, serine, threonine, tyrosine, cysteine),
- nonpolar side chains (e.g., alanine, valine, leucine, isoleucine, proline, phenylalanine, methionine, tryptophan),
- beta-branched side chains (e.g., threonine, valine, isoleucine) and
- aromatic side chains (e.g., tyrosine, phenylalanine, tryptophan, histidine).

Other conserved amino acid substitutions can also occur across amino acid side chain families, such as when substituting an asparagine for aspartic acid in order to modify the charge of a peptide. Thus, a predicted nonessential amino acid residue in a HR domain polypeptide, for example, is preferably replaced with another amino acid residue from the same side chain family or homologues across families (e.g. asparagine for aspartic acid, glutamine for glutamic acid). Conservative changes can further include substitution of chemically homologous non-natural amino acids (i.e. a synthetic non-natural hydrophobic amino acid in place of leucine, a synthetic non-natural aromatic amino acid in place of tryptophan).

According embodiments of the invention, the second binding domain which binds to CD3 comprises the CDRs and/or variable domains of an antibody selected from the group consisting of OKT3 or BlinCD3 as well as, UCHT-1 (Ceuppens et al, 1986), BMA031 (Borst et al, 1990) and 12F6 (Li et al, 2005).

The anti CD3 antibody OKT3 is disclosed in Horn et al. (2017) the content of which is incorporated herein by reference. The anti CD3 antibody BlinCD3 is a modified variant of OKT3.

According to one embodiment of the invention, the second binding domain which binds to CD3
a) comprises a set of heavy chain/light chain complementarity determining regions (CDR) comprised in the heavy chain/light variable region sequence pair of one of the anti CD3 antibodies BlinCD3 and OKT3, as set forth in SEQ ID NOs 1 and 8, or SEQ ID NOs 16 and 20, respectively
b) comprises the set of heavy chain/light chain complementarity determining regions (CDR) comprising the following sequences
   - HC CDR1 (SEQ ID NO 2 or 5)
   - HC CDR2 (SEQ ID NO 3 or 6)
   - HC CDR3 (SEQ ID NO 4 or 7)
   - LC CDR1 (SEQ ID NO 9 or 12)
   - LC CDR2 (SEQ ID NO 10 or 13), and
   - LC CDR3 (SEQ ID NO 11 or 14)
c) comprises the set of heavy chain/light chain complementarity determining regions (CDR) comprising the following sequences
   - HC CDR1 (SEQ ID NO 17)
   - HC CDR2 (SEQ ID NO 18)
   - HC CDR3 (SEQ ID NO 19)
   - LC CDR1 (SEQ ID NO 21)
   - LC CDR2 (SEQ ID NO 22), and
   - LC CDR3 (SEQ ID NO 23)
d) comprises the heavy chain/light chain complementarity determining regions (CDR) of b) or c), with the proviso that at least one of the CDRs has up to 3 amino acid substitutions relative to the respective SEQ ID NO 2 - 4, 9 - 11, 17 - 19 or 21 - 23, and/or
e) comprises the heavy chain/light chain complementarity determining regions (CDR) of b), with the proviso that at least one of the CDRs has a sequence identity of ≥ 66 % to the respective SEQ ID NO 2 - 4, 9 - 11, 17 - 19 or 21 - 23,
wherein the CDRs are embedded in a suitable protein framework so as to be capable to bind to CD3.

With regard to item b), two different sets of CDRs are disclosed, because the counting of CDRs may vary from one counting method to the other (e.g., Kabat, Chothia or MacCallum).

According to one embodiment of the invention, the second binding domain which binds to CD3 comprises
a) the variable domains of one antibody selected from the group consisting of OKT3 and BlinCD3
b) the heavy chain/light chain variable domains (VD)
   - HC VD (SEQ ID NO 1 or 16), and
   - LC VD (SEQ ID NO 8 or 20)
c) the heavy chain/light chain variable domains (VD) of b), with the proviso that
   - the HCVD has a sequence identity of ≥ 80 % to the respective SEQ ID NO 1 or 16, and/or
   - the LCDVD has a sequence identity of ≥ 80 % to the respective SEQ ID NO 8 or 20
d) the heavy chain/light chain variable domains (VD) of b), with the proviso that at least one of the HCVD or LCVD has up to 10 amino acid substitutions relative to the respective SEQ ID NOs,
wherein said bispecific binding agent is still capable to bind to CD3.

According to one embodiment, the framework is a human VH/VL framework. According to one embodiment, at least one amino acid substitution is a conservative amino acid substitution.

According to one embodiment of the bispecific binding agent, the second binding domain which binds to CD3
a) comprises a scFv-like sequence as set forth in any one of SEQ ID NOs 15 or 24, or
b) comprises an amino acid sequence that has a sequence identity of ≥ 80 % to any one of SEQ ID NOs 15 or 24.

According to one embodiment of the bispecific binding agent, the first binding domain which binds to Fluorescein or conjugated Fluorescein
a) comprises a scFv-like sequence as set forth in any one of SEQ ID NOs 35 or 44, or
b) comprises an amino acid sequence that has a sequence identity of ≥ 80 % to any one of SEQ ID NOs 35 or 44.

According to one embodiment of the bispecific binding agent, the first binding domain which binds to Fluorescein or conjugated Fluorescein
a) comprises at least one of the sequences selected from SEQ ID NOs 48 - 50, or
b) comprises an amino acid sequence that has a sequence identity of ≥ 80 % to SEQ ID NOs 48 - 50,
with optionally a C terminal cysteine residue and/or a His tag removed or in place.

According to one embodiment of the bispecific binding agent, the agent has at least one of
- target binding affinity of ≥ 50 % to CD3, and measured by SPR, and/or
- target binding affinity of ≥ 50 % to Fluorescein or conjugated Fluorescein, and measured by SPR,
compared to that of the bispecific binding agent according to the above description.

According to another aspect of the invention, a bispecific binding agent is provided which competes for binding to Fluorescein or conjugated Fluorescein, and/or CD3, respectively, with the bispecific binding agent according to the above description.

According to another aspect of the invention, a nucleic acid is provided which encodes for a binding agent according to the above description. Such nuclei acid can be an mRNA, a cDNA, a DNA or a genomic DNA, which can comprised non coding stretches (Introns). Due to the degeneracy of the gentic code, a large number of different nucleic acid sequences can enclose for the same binding agent. However, based on the sequence information of the binding agent the skilled person can determine or identify one or more such nucleic acid molecules.

According to another aspect of the invention, a combination is provided comprising
a) the bispecific binding agent according to the above description, and
b) a labelled binding agent that binds specifically to a target antigen,
wherein the labelled binding agent is labelled with an organic fluorophore that is specifically detected by the first binding domain of the binding agent according to the above description.

According to another aspect of the invention, a dosage regimen is provided, comprising administration to a patient, consecutively or concomitantly.
of
a) the bispecific binding agent according to the above description, and
b) a labelled binding agent that binds specifically to a target antigen,
wherein the labelled binding agent is labelled with an organic fluorophore that is specifically detected by the first binding domain of the binding agent according to the above description.

The labelled binding agent is also called "adaptor molecule" or "adaptor" herein, as it provides target specificity to the combination. Technically, the labelled binding agent can be chosen from large libraries of binding agents, including antibodies, that are specific to every conceivable target antigen.

In one embodiment, the target antigen is an antigen that is presented by tumor cells of a hematological tumor. In one embodiment, the target antigen is an antigen that is presented by tumor cells of a solid tumor.

The advantages of using an organic fluorophore in the adaptor molecule are manifold:
a) for many organic fluorophores commercially available antibodies with high affinity exist that can be used in the bispecific binding agent
b) organic fluorophores are relatively small (fluorescein has e.g. a molecular weight of about 390 Da)
c) organic fluorophores are non-immunogenic and non-toxic (fluorescein is being used in eye drops for example)
d) a binding agent that is labelled with an organic fluorophore can be monitored e.g. in preclinical studies
e) proven chemistry exists to stably conjugate organic fluorophores to binding agents like e.g. antibodies, avoiding dissociation of the organic fluorophore from the binding agent in the bloodstream (as could happen for peptide labels).

As regards the bispecific binding agent in the said combination, the same embodiments apply as disclosed above for the bispecific binding agent alone.

According to one embodiment of the combination or dosage regimen according to the above description, the bispecific binding agent according and the labelled binding agent are administered individually, or are first mixed with one another and then administered.

According to one embodiment of the combination or dosage regimen according to the above description, the organic fluorophore in the labelled binding agent is fluorescein.

As used herein, the terms "Fluorescein", "Fluorescein isothiocyanate" and "FITC" are being used interchangeably.

According to one embodiment of the combination or dosage regimen according to the above description, the labelled binding agent is site-specifically labelled with the organic fluorophore.

In such way, it can be assured that
a) a constant stochimetry ratio between binding agent and organic fluorophore can be achieved, which is crucial for a reproducible dosage of the combination of bispecific binding agent and labelled binding agent.
b) the labelling does not interfere with the binding of the labelled bispecific agent to its target.

In one embodiment, a site specific labeling with fluorescein ("FITCylation" can be obtained by the use of (i) fluorescein-5-maleimide (5-MF), and (ii) a proteinaceous binding agent - like an antibody, fragment or derivative thereof, that has a C-terminal cysteine.

Such approach is disclosed in the experimental section herein, and was for example described in Pellegrino et al. 2020), the content of which is incorporated herein by reference for enablement purposes.

According to one embodiment of the combination or dosage regimen according to the above description, the labelled binding agent comprises an antibody, or a target binding fragment or derivative thereof.

In a preferred embodiment, the labelled binding agent is at least one selected from the group consisting of an
- antibody,
- antibody-based binding protein
- modified antibody format retaining target binding capacity,
- antibody derivative or fragment retaining target binding capacity, and/or
- antibody mimetic.

As used herein, the term "binding agent" is meant to define an entity, an agent or a molecule that has affinity to a given target, e.g., a receptor, a cell surface protein, a cytokine or the like. Such Binding agent may optionally block or dampen agonist-mediated responses, or inhibit receptor-agonist interaction. Most importantly, however, the binding agent may serve as a shuttle to deliver a payload to a specific site, which is defined by the target recognized by said binding agent. Thus, a binding agent targeting, for instance, but not limited to a receptor, delivers its payload to a site which is characterized by abundance of said receptor.

Binding agent s include, but are not limited to, antibodies, antibody fragments, antibody-based binding proteins, antibody mimetics, receptors, soluble decoy receptors, scaffold proteins with affinity for a given target and ligands of receptors.

"Antibodies", also synonymously called "immunoglobulins" (Ig), are generally comprising four polypeptide chains, two heavy (H) chains and two light (L) chains, and are therefore multimeric proteins, or an equivalent Ig homologue thereof (e.g., a camelid antibody, which comprises only a heavy chain, single domain antibodies (dAbs) which can be either be derived from a heavy or light chain); including full length functional mutants, variants, or derivatives thereof (including, but not limited to, murine, chimeric, humanized and fully human antibodies, which retain the essential epitope binding features of an Ig molecule, and including dual specific, bispecific, multispecific, and dual variable domain immunoglobulins; Immunoglobulin molecules can be of any class (e.g., IgG, IgE, IgM, IgD, IgA, and IgY), or subclass (e.g., IgG1, IgG2, IgG3, IgG4, IgAl, and IgA2) and allotype.

An "antibody-based binding protein", as used herein, may represent any protein that contains at least one antibody-derived V_{H}, V_{L}, or C_{H} immunoglobulin domain in the context of other non-immunoglobulin, or non-antibody derived components. Such antibody-based proteins include, but are not limited to (i) F_{c}-fusion proteins of binding proteins, including receptors or receptor components with all or parts of the immunoglobulin C_{H} domains, (ii) binding proteins, in which V_{H} and or V_{L} domains are coupled to alternative molecular scaffolds, or (iii) molecules, in which immunoglobulin V_{H}, and/or V_{L}, and/or C_{H} domains are combined and/or assembled in a fashion not normally found in naturally occurring antibodies or antibody fragments.

An "antibody derivative or fragment", as used herein, relates to a molecule comprising at least one polypeptide chain derived from an antibody that is not full length, including, but not limited to (i) a Fab fragment, which is a monovalent fragment consisting of the variable light (V_{L}), variable heavy (V_{H}), constant light (C_{L}) and constant heavy 1 (C_{H}I) domains; (ii) a F(ab')2 fragment, which is a bivalent fragment comprising two Fab fragments linked by a disulfide bridge at the hinge region; (iii) a heavy chain portion of a Fab (Fa) fragment, which consists of the V_{H} and C_{H}I domains; (iv) a variable fragment (Fᵥ) fragment, which consists of the V_{L} and V_{H} domains of a single arm of an antibody, (v) a domain antibody (dAb) fragment, which comprises a single variable domain; (vi) an isolated complementarity determining region (CDR); (vii) a single chain Fᵥ Fragment (scFᵥ); (viii) a diabody, which is a bivalent, bispecific antibody in which V_{H} and V_{L} domains are expressed on a single polypeptide chain, but using a linker that is too short to allow for pairing between the two domains on the same chain, thereby forcing the domains to pair with the complementarity domains of another chain and creating two antigen binding sites; (ix) a linear antibody, which comprises a pair of tandem Fᵥ segments (V_{H}-C_{H}1-V_{H}-C_{H}1) which, together with complementarity light chain polypeptides, form a pair of antigen binding regions; (X) Dual-Variable Domain Immunoglobulin (xl) other non-full length portions of immunoglobulin heavy and/or light chains, or mutants, variants, or derivatives thereof, alone or in any combination.

The term "modified antibody format", as used herein, encompasses antibody-drug- conjugates, Polyalkylene oxide-modified scFv, Monobodies, Diabodies, Camelid Antibodies, Domain Antibodies, bi- or trispecific antibodies, IgA, or two IgG structures joined by a J chain and a secretory component, shark antibodies, new world primate framework + non-new world primate CDR, IgG4 antibodies with hinge region removed, IgG with two additional binding sites engineered into the CH3 domains, antibodies with altered Fc region to enhance affinity for Fc gamma receptors, dimerised constructs comprising CH3+VL+VH, and the like.

The term "antibody mimetic", as used herein, refers to proteins not belonging to the immunoglobulin family, and even non-proteins such as aptamers, or synthetic polymers. Some types have an antibody-like beta-sheet structure. Potential advantages of "antibody mimetics" or "alternative scaffolds" over antibodies are better solubility, higher tissue penetration, higher stability towards heat and enzymes, and comparatively low production costs.

Some antibody mimetics can be provided in large libraries, which offer specific binding candidates against every conceivable target. Just like with antibodies, target specific antibody mimetics can be developed by use of High Throughput Screening (HTS) technologies as well as with established display technologies, just like phage display, bacterial display, yeast or mammalian display. Currently developed antibody mimetics encompass, for example, ankyrin repeat proteins (called DARPins), C-type lectins, A- domain proteins of S. aureus, transferrins, lipocalins, 10th type III domains of fibronectin, Kunitz domain protease inhibitors, ubiquitin derived binders (called affilins), gamma crystallin derived binders, cysteine knots or knottins, thioredoxin A scaffold based binders, nucleic acid aptamers, artificial antibodies produced by molecular imprinting of polymers, peptide libraries from bacterial genomes, SH-3 domains, stradobodies, "A domains" of membrane receptors stabilised by disulfide bonds and Ca2+, CTLA4-based compounds, and Fyn SH3.

In one embodiment, the labelled binding agent is a scfV fragment, for example having one of the following structures:
- VL₁-VH₁
- VH₁-VL₁

In one embodiment, the labelled binding agent is a diabody, for example having one of the following structures:
- VL₁-VH₁-VL₁-VH₁
- VH₁-VL₁-VH₁-VL₁

According to embodiments of the combination or dosage regimen according to the above description, the labelled binding agent binds specifically to a target antigen selected from the group consisting of CD117 and CAIX.

Preferably, CD117/c-KIT is human CD117/c-KIT as e.g. disclosed under UniProt identifier P10721. Mast/stem cell growth factor receptor (SCFR), also known as proto-oncogene c-KIT or tyrosine-protein kinase KIT or CD117, is a receptor tyrosine kinase protein that in humans is encoded by the KIT gene. Multiple transcript variants encoding different isoforms have been found for this gene. KIT was first described by the German biochemist Axel Ullrich in 1987 as the cellular homolog of the feline sarcoma viral oncogene v-kit.

CD117 is a cytokine receptor expressed on the surface of hematopoietic stem cells as well as other cell types. Altered forms of this receptor may be associated with some types of cancer. CD117 is a receptor tyrosine kinase type III, which binds to stem cell factor (a substance that causes certain types of cells to grow), also known as "steel factor" or "c-kit ligand". When this receptor binds to stem cell factor (SCF) it forms a dimer that activates its intrinsic tyrosine kinase activity, that in turn phosphorylates and activates signal transduction molecules that propagate the signal in the cell. After activation, the receptor is ubiquitinated to mark it for transport to a lysosome and eventual destruction. Signaling through CD117 plays a role in cell survival, proliferation, and differentiation. For instance, CD117 signaling is required for melanocyte survival, and it is also involved in haematopoiesis and gametogenesis.

Like other members of the receptor tyrosine kinase III family, CD117 consists of an extracellular domain, a transmembrane domain, a juxtamembrane domain, and an intracellular tyrosine kinase domain. The extracellular domain is composed of five immunoglobulin-like domains, and the protein kinase domain is interrupted by a hydrophilic insert sequence of about 80 amino acids. The ligand stem cell factor binds via the second and third immmunoglobulin domains.

Cluster of differentiation (CD) molecules are markers on the cell surface, as recognized by specific sets of antibodies, used to identify the cell type, stage of differentiation and activity of a cell. CD117 is an important cell surface marker used to identify certain types of hematopoietic (blood) progenitors in the bone marrow. To be specific, hematopoietic stem cells (HSC), multipotent progenitors (MPP), and common myeloid progenitors (CMP) express high levels of CD117. Common lymphoid progenitors (CLP) express low surface levels of CD117. CD117 also identifies the earliest thymocyte progenitors in the thymus-early T lineage progenitors (ETP/DN1) and DN2 thymocytes express high levels of c-Kit. It is also a marker for mouse prostate stem cells. In addition, mast cells, melanocytes in the skin, and interstitial cells of Cajal in the digestive tract express CD117. In humans, expression of c-kit in helper-like innate lymphoid cells (ILCs) which lack the expression of CRTH2 (CD294) is used to mark the ILC3 population.

In humans, CD117/c-Kit is *inter alia* present in hematopoietic cells and plays a crucial role in early stages of hematopoiesis, but is also expressed in AML blasts

Preferably CAIX (Carbonic anhydrase IX, also called CA09) is human CAIX (UniProt identifier: A0A087WYS4. Carbonic anhydrase IX (CAIX) is a suitable target for various anticancer strategies. It is a cell surface protein that is present in human tumors, but not in the corresponding normal tissues. Expression of CAIX is induced by hypoxia and correlates with cancer prognosis in many tumor types. Moreover, CAIX is functionally implicated in cancer progression as a pro-survival factor protecting cancer cells against hypoxia and acidosis via its capability to regulate pH and cell adhesion.

Cancer-related distribution of CAIX allows for targeting cancer cells by antibodies binding to its extracellular domain, whereas functional involvement of CAIX opens the possibility to hit cancer cells by blocking their adaptation to physiologic stresses via inhibition of CAIX enzyme activity. The latter strategy is recently receiving considerable attention and great efforts are made to produce CAIX-selective inhibitor derivatives with anticancer effects.

Targeting CAIX-expressing cells by immunotherapy has reached clinical trials and is close to application in treatment of renal cell carcinoma patients. Nevertheless, development and characterization of new CAIX-specific antibodies is still ongoing.

According to one embodiment of the combination or dosage regimen according to the above description, the labelled binding agent that binds to CD117
a) comprises a set of heavy chain/light chain complementarity determining regions (CDR) comprised in the heavy chain/light variable region sequence pair of the anti CD117 antibody 79D, as set forth in SEQ ID NOs 52 and 59,
b) comprises the set of heavy chain/light chain complementarity determining regions (CDR) comprising the following sequences
   - HC CDR1 (SEQ ID NO 53 or 56)
   - HC CDR2 (SEQ ID NO 54 or 57)
   - HC CDR3 (SEQ ID NO 55 or 58)
   - LC CDR1 (SEQ ID NO 60 or 63)
   - LC CDR2 (SEQ ID NO 61 or 64), and
   - LC CDR3 (SEQ ID NO 62 or 65)
c) comprises the heavy chain/light chain complementarity determining regions (CDR) of b) or c), with the proviso that at least one of the CDRs has up to 3 amino acid substitutions relative to the respective SEQ ID NO 53 - 55, 60 - 62, 56 - 57 or 63 - 65, and/or
d) comprises the heavy chain/light chain complementarity determining regions (CDR) of b), with the proviso that at least one of the CDRs has a sequence identity of ≥ 66 % to the respective SEQ ID NO 53 - 55, 60 - 62, 56 - 57 or 63 - 65,
wherein the CDRs are embedded in a suitable protein framework so as to be capable to bind to CD117

The anti CD117 antibody 79D is disclosed in X, the content of which is incorporated herein by reference.

With regard to item b), two different sets of CDRs are disclosed, because the counting of CDRs may vary from one counting method to the other (e.g., Kabat, Chothia or MacCallum).

According to one embodiment of the combination or dosage regimen according to the above description, the labelled binding agent that binds to CD117 comprises
a) the variable domains of the antibody 79D
b) the heavy chain/light chain variable domains (VD)
   - HC VD (SEQ ID NO 52), and
   - LC VD (SEQ ID NO 59)
c) the heavy chain/light chain variable domains (VD) of b), with the proviso that
   - the HCVD has a sequence identity of ≥ 80 % to the respective SEQ ID NO 52, and/or
   - the LCDVD has a sequence identity of ≥ 80 % to the respective SEQ ID NO 59
d) the heavy chain/light chain variable domains (VD) of b), with the proviso that at least one of the HCVD or LCVD has up to 10 amino acid substitutions relative to the respective SEQ ID NOs,
wherein said labelled binding agent is still capable to bind to CD117.

With regard to the different alternatives disclosed, the same embodiments apply as with regard to the bispecific binding agent disclosed elsewhere herein, in particular regarding the homology ranges and the conservative amino acid substitutions.

With regard to the different alternatives disclosed, the same embodiments apply as with regard to the bispecific binding agent disclosed elsewhere herein, in particular regarding the homology ranges and the conservative amino acid substitutions

According to another aspect of the invention, a pharmaceutical composition comprising the combination according the above description is provided, which optionally comprises one or more pharmaceutically acceptable excipients.

According to one aspect of the invention, the combination or dosage regimen or pharmaceutical composition according to the above description is provided for use (in the manufacture of a medicament) in the treatment of a human or animal subject
- being diagnosed for,
- suffering from or
- being at risk of
developing a neoplastic disease, or for the prevention of such condition.

In preferred embodiments, such neoplastic disease is a solid tumor or a haematological tumor. The examples provided in the present specification provided enabling support for both fields of application.

According to one aspect of the invention, a method for treating or preventing a neoplastic disease is provided, comprising administering to a subject in need thereof an effective amount of the combination or pharmaceutical composition according to the above description, or subjecting said patient to dosage regimen according to the above description.

According to one aspect of the invention, a therapeutic kit of parts is provided, comprising:
a) the combination or pharmaceutical composition according to the above description
b) an apparatus for administering the combination or composition, and
c) optionally, instructions for use.

### Examples

While the invention has been illustrated and described in detail in the drawings and foregoing description, such illustration and description are to be considered illustrative or exemplary and not restrictive; the invention is not limited to the disclosed embodiments. Other variations to the disclosed embodiments can be understood and effected by those skilled in the art in practicing the claimed invention, from a study of the drawings, the disclosure, and the appended claims. In the claims, the word "comprising" does not exclude other elements or steps, and the indefinite article "a" or "an" does not exclude a plurality. The mere fact that certain measures are recited in mutually different dependent claims does not indicate that a combination of these measures cannot be used to advantage. Any reference signs in the claims should not be construed as limiting the scope.

All amino acid sequences disclosed herein are shown from N-terminus to C-terminus; all nucleic acid sequences disclosed herein are shown 5'->3'.

### Materials and Methods

### Cell lines

NALM-6 (ACC 128, DSMZ) and MOLM-14 (ACC 777, DSMZ) cells were cultured in RPMI-1640 supplemented with 20% FBS and 1% penicillin/streptomycin. Cell lines were expanded and stored as cryopreserved aliquots in liquid nitrogen. Cells were grown according the supplier's protocol and kept in culture for no longer than 2 weeks. Authentication of the cell line also included check of post-freeze viability, growth properties, morphology, test for mycoplasma contamination, isoenzyme assay, and sterility test were performed by the cell bank before shipment. MOLM-14 cells were lentivirally transduced and subsequently sorted to homogeneously express CD117 on their surface (Myburgh et al. 2020).

### Isolation and purification of T-cells

Healthy donors' buffy coats were acquired from the Zurich blood donation service (Blutspende Zürich, Zürich, Switzerland). Peripheral blood mononuclear cells (PBMCs) were isolated by density gradient centrifugation on Ficoll Paque Plus (GE Healthcare) according to the manufacturer's instructions. T-cells were then negatively isolated with EasySep^{™} beads (Human T cell isolation kit, STEMCELL Technologies). Purified T-cell samples were resuspended in 90% FCS supplemented with 10% DMSO and cryo-preserved at -80°C and subsequently in liquid nitrogen.

### Cloning of UniTE(4m5.3/BlinCD3), UniTE(E2/BlinCD3) and UniTE(FluA/BlinCD3)

The sequences of a humanized version of the anti-human CD3 antibody OKT3, called Blin CD3 (SEQ ID NOs 1-14) and the sequences of the anti-fluorescein murine 4m5.3 (SEQ ID NOs 36-34) or human E2 antibody (27.35) (disclosed in Boder et al. (2000) and Vaughan et al. (1996)), or anticalin^{™} FluA (disclosed in Vopel et al. (2005) (SEQ ID NO 47) were genetically assembled by PCR in the following formats to obtain the UniTEs 4m5.3/BlinCD3, FluA/ BlinCD3 and E2/BlinCD3 (lower row shows the SEQ ID NOs):

| VL (anti-FITC) | Linker15aa | VH (anti-FITC) | Linker5aa | VH (BlinCD3) | Linker18aa | VL (BlinCD3 | 6xHis | Total |
|---|---|---|---|---|---|---|---|---|
| 40 | 45 | 36 | 67 | 1 | 26 | 8 | HHHHHH | 49 |

| FluA | Linker 5aa | VH (BlinCD3 | Linker 18aa | VL (BlinCD3 | 6xHis | Total |
|---|---|---|---|---|---|---|
| 47 | 67 | 1 | 26 | 8 | HHHHHH | 50 |

| VL (anti-FITC) | Linker15aa | VH (anti-FITC) | Linker5aa | VH (BlinCD3 | Linker18aa | VL (BlinCD3 | 6xHis | Total |
|---|---|---|---|---|---|---|---|---|
| 27 | 45 | 31 | 67 | 1 | 26 | 8 | HHHHHH | 48 |

And cloned into the mammalian expression vector pcDNA3.1(+) (Invitrogen).

### Cloning of antibodies that to be used as adaptors

Two different labelled binding agents ("adaptors") were created, both of which are antibodies. One adaptor binds CD117, which, as discussed elsewhere herein, is a target expressed on blood cells and hematopoietic cells. It hence stands representative for haematological cancers.

The other adaptor binds to CAIX (Carbonic anhydrase IX, also called CA09), which as discussed elsewhere herein, is a cell surface protein that is present in human tumors, but not in the corresponding normal tissues. It hence stands representative for solid tumors.

The anti CD117 antibody is described in Reshetnyak et al. (2013), and bears the clonal name antibody 79D. It comprises the heavy chain/light chain variable domain HC VD (SEQ ID NO 52), and LC VD (SEQ ID NO 59).

The anti CAIX antibody is described in Cazzamalli et al (2018), and bears the clonal name antibody XE114. It comprises the heavy chain/light chain variable domain HC VD (SEQ ID NO 68), and LC VD (SEQ ID NO 72).

### anti-CD117 antibody

Sequences of the variable region of the light and heavy chains of the anti-CD117 antibody were cloned in a diabody (Db) format (VL-Linker 5aa -VH) with a C-terminal poly-histidine tag into pcDNA3.1(+). An additional C-terminal cysteine was added for site-selective conjugations.

### anti-CAIX antibody

The anti CAIX antibody was cloned and prepared as an IgG as described in Cazzamalli et al (2018), the content of which is incorporated herein by reference.

### Expression of antibodies

The antibodies were produced by transient gene expression using polyethyleneimine in CHO-S cells (Invitrogen) following standard protocols⁸. The proteins were purified by Ni-NTA agarose resin (ThermoFisher) and analyzed using SDS-PAGE and size exclusion chromatography (Superdex 200 Increase, 10/300, GE Healthcare).

### Conjugation of anti-CD117 and anti-CAIX to fluorescein

Antibody solutions in PBS (1 mg/ml) were reduced with 50 eq. of DTT (1,4-Dithio-DL-threitol) in PBS. After reduction, the protein solution was washed three times with PBS using Vivaspin centrifugation column (Sartorius) to dilute the DTT. Fluorescein-5-maleimide (Thermo Scientific) was dissolved in DMSO anhydrous to obtain a final concentration of 10% (v/v) when added to the reduced protein. Antibodies were treated with 50 eq. per cysteine of fluorescein, and homogeneous conjugates with single modification within the light chain was observed by LC-MS after 1hr. Conjugated proteins were purified by PD-10 desalting columns (GE Healthcare) and eluted in Acetate Buffer, pH 5. Aliquots were snap-frozen in liquid nitrogen and stored at -80°C until further use.

### In vitro UniTE-mediated T-cell killing of CD19⁺ tumor cells

Healthy donor's T cells were thawed 1 day before the assay and cultured overnight in advanced RPMI supplemented with 1x Glutamax (Gibco), 10% FBS and 1% penicillin/streptomycin (T cell medium). T cells and CD19+ B acute lymphoblastic leukemia (B-ALL) NALM-6 cells were co-incubated at 10:1 ratio (E:T=10:1) in presence of 10nM of a commercially available FITC-anti-human CD19 mouse IgG1 (Biolegend; clone HIB19). UniTE(4m5.3/BlinCD3) and UniTE(FluA/BlinCD3) were compared with the clinically approved (anti-CD 19/BlinCD3) bispecific T cell engager (BiTE^{™}) Blinatumomab (Blycinto^{™}) at the same concentrations (100nM and 10nM). The specific lysis of target cells was analyzed by flow cytometry using LSR II Fortessa cell analyzer (BD Biosciences) and quantified using the following formula: (1-number of alive target cells/ number of alive target cells without antibody)^{∗}100.

### In vitro UniTE-mediated T-cell killing of CD117⁺ tumor cells

T cells were thawed 1 day before the assay and cultured overnight in T cell medium. T cells and acute myeloid leukemia (AML) MOLM-14 cells (transduced to express CD117 on the surface) were co-cultured in T cell medium (E:T = 1:1) in presence of 10nM of anti CD117 diabody site-specifically conjugated with fluorescein. UniTE(4m5.3/BlinCD3) killing was compared with an anti-CD 117/BlinCD3 bispecific T cell engager at equimolar concentrations (100nM and InM) and with direct anti-CD117 CAR-T cells¹, both bearing the scFv from the same clone of the bridging Db. The specific lysis of target cells was analyzed as previously described.

### In vitro UniTEA-mediated T-cell killing of CAIX⁺ tumor cells

T cells were thawed 1 day before the assay and cultured overnight in T cell medium. CAIX⁺ renal cell carcinoma SK-RC-52 cells were harvested 1 day before the assay and membrane stained using PKH26 Red Fluorescent Cell Linker Kit for General Membrane (Sigma-Aldrich) according to manufacturer instructions. Stained SK-RC-52 cells were seeded in a 96 well plate and incubated overnight. The next day, SK-RC-52 cells and T cells were co-cultured in T cell medium (E:T = 10:1) in presence of 10nM site-specifically fluoresceinated anti-CAIX IgG and different concentration of 4m5.3/BlinCD3, FluA/BlinCD3 and E2/BlinCD3 UniTEAs (1, 10 and 100nM). After 24hrs, the specific lysis of target cells was analyzed as previously described. Background killing after co-culturing T- and tumor cells alone or by adding only 10nM adaptor molecule was also analyzed.

### In vivo stability of fluorescein molecules on antibodies

8-week old C57BL/6J mice were i.v. injected with 50ug of anti-murine CD19-FITC antibodies (Biolegend; clone 1D3). Murine B cells were analyzed by flow cytometry from peripheral blood at 4hrs, 1 day, 4 days and 8 days post injection. The peripheral blood was lysed for 10min with RBC lysis reagent (Biolegend) and washed 1x with PBS (Invitrogen). Each sample was split in 2 and stained with only anti-mouse CD20 (Biolegend; clone SA271G2) or with anti-mouse CD20 (Biolegend; clone SA271G2) and anti-mouse CD19-FITC (Biolegend; clone 1D3). Samples were stained in FACS buffer (PBS with 2% FBS and 2mM EDTA) and washed twice after incubation prior to FACS analysis.

### Statistical Analysis

Data are reported as mean ± standard deviation (SD) unless otherwise noted. Statistical analysis was performed GraphPad Prism 8.0 software. FlowJo software (v10.0.7, FlowJo) was used for data analysis and presentation.

### Results

### Production of various UniTEA formats

Aiming to re-direct, activate and induce T-cell mediated killing of potentially any target cell *via* a fluorescein-tagged target-binding molecules [Figure 1A], we generated 3 versions of the UniTEs which all bind human CD3ε and fluorescein [Figure 1B]. The 3 versions are designated as follows; 4m5.3/BlinCD3, E2/BlinCD3 and FluA/BlinCD3. Each UniTEA version differs in the fluorescein-binding moiety. 4m5.3 and E2 are antibody fragments in scFv format against fluorescein of mouse and human origin, respectively. FluA is a lipocalin fluorescein-binding molecule derived from *Pieris brassicae.*

UniTEs redirect T-cells to deplete CD19 and CD117-expressing leukemia cell lines *in vitro* Two UniTEs - 4m5.3/BlinCD3 and FluA/BlinCD3 - were tested functionally *in vitro* by determining their potential to redirect T cell killing against CD19⁺ and CD117⁺ human cell lines [Figure 2]. The CD19⁺ NALM6 cell line was used to compare the T-cell mediated killing elicited by the 4m5.3/BlinCD3 and FluA/BlinCD3 UniTEA formats compared to the clinical-grade BiTE^{™} Blinatumomab (anti-CD 19/BlinCD3) [Figure 2A]. We confirm that T-cell activation and killing is elicited only in the presence of both the UniTEA and adaptor molecule since target cells and T-cells co-incubated with either UniTEA or bridge molecule alone led to minimal (∼5%) background lysis of target cells [Figure 2B]. When using an effector to target ratio as high as 10:1, it is expected to see some spontaneous activity of the T cells against target cells in the absence and any T cell activating agent. After 24 hours, Blinatumomab resulted in over 50% and up to 77% T-cell mediated target cell lysis [Figure 2C]. The addition of 4m5.3/BlinCD3 or FluA/BlinCD3 UniTEA could achieve similar target cell lysis of equimolar concentration of Blinatumomab when 10nM of commercially available anti-CD 19 IgG-FITC (Biolegend; clone HIB1) was used as bridging molecule [Figure 2C]. The CD117⁺ MOLM14 cell line was used to compare the T-cell mediated killing elicited by the 4m5.3/BlinCD3 and FluA/BlinCD3 UniTEA formats compared to conventional CAR-T cells and BiTE^{™} directed against CD117 [Figure 2D]. The CD117 targeting CAR T cell and BiTE^{™} could achieve >95% target cell lysis after 24 hours [Figure 2E]. Similar target cell lysis was exhibited at the same time point when 100nM of 4m5.3/BlinCD3 UniTEA were added in combination with 10nM fluorescein-labeled anti-CD117 Db. The addition of 1nM of 4m5.3/BlinCD3 UniTEA resulted in >95% target cell lysis after 48 hours [Figure 2E].

### In vivo persistence of fluoresceinated antibodies

The stability in blood of the fluorescein over time is necessary for an efficient UniTE-fluoresceinated bridge complex formation and T-cell redirection. In order to assess the availability of structurally intact fluorescein on bridge molecules we administered 50ug of commercially available anti-murine CD19 (mCD19) IgG-FITC and analyzed the peripheral blood at different time points spanning 8 days [Figure 3A]. At each time point analyzed, mCD19⁺ cells belonging to same mouse showed no difference in mean fluorescence intensity (MFI) when stained with or without additional mCD19-FITC [Figure 3B]. However, the MFI of the mCD19⁺ population decreased after 24 hours but then remained stable until day 8 [Figure 3B]. There was no difference in the proportion of mCD19⁺/mCD20⁺ B cells (expressed as percentage of mCD19⁺/mCD20⁺ cells compared to the baseline number after 4hrs) stained with and without additional CD19-FITC labelling [Figure 3C]. Since additional CD19-FITC labelling for flow cytometry did not increase the proportion of CD19⁺ cells or the MFI, all available CD19 epitopes were still occupied by the injected CD19-FITC antibody at day 0 and FITC remains stable in vivo over 5 days. Only at day 8, additional mCD19-FITC labelling resulted in an increase of the MFI of the CD19⁺ population indicating that at day 8 some of the injected mCD19-FITC antibody had been cleared from the body. Representative FACS plots showing the peripheral blood mCD19⁺/mCD20⁺ B cell population at indicated time points with and without additional mCD19-FITC labelling are reported in Figure 3D. These populations were pre-gated on live single cells.

### References

Myburgh, R. et al. Anti-human CD117 CAR T-cells efficiently eliminate healthy and malignant CD117-expressing hematopoietic cells. Leukemia (2020).
Horn, L. A. et al. CD3xPDL1 bi-specific T cell engager (BiTE) simultaneously activates T cells and NKT cells, kills PDL1+ tumor cells, and extends the survival of tumor-bearing humanized mice. Oncotarget 8, 57964 (2017).
Boder, E. T., Midelfort, K. S. & Wittrup, K. D. Directed evolution of antibody fragments with monovalent femtomolar antigen-binding affinity. Proc. Natl. Acad. Sci. 97, 10701-10705 (2000).
Vaughan, T. J. et al. Human Antibodies with Sub-nanomolar Affinities Isolated from a Large Non-immunized Phage Display Library. Nat. Biotechnol. 14, 309-314 (1996).
Vopel, S., Mühlbach, H. & Skerra, A. Rational engineering of a fluorescein-binding anticalin for improved ligand affinity. Biol. Chem. 386, 1097-1104 (2005).
Reshetnyak, A. V. et al. Structural basis for KIT receptor tyrosine kinase inhibition by antibodies targeting the D4 membrane-proximal region. Proc. Natl. Acad. Sci. U. S. A. 110, 17832-17837 (2013).
Casi, G., Huguenin-Dezot, N., Zuberbühler, K., Scheuermann, J. & Neri, D. Site-specific traceless coupling of potent cytotoxic drugs to recombinant antibodies for pharmacodelivery. J. Am. Chem. Soc. 134, 5887-5892 (2012).
Rajendra, Y., Kiseljak, D., Baldi, L., Hacker, D. L. & Wurm, F. M. A simple high-yielding process for transient gene expression in CHO cells. J. Biotechnol. 153, 22-26 (2011).
Plückthun and Skerra, Meth. Enzymol., 178:497-515 (1989) and in Day, E. D., Advanced Immunochemistry, Second Ed., Wiley-Liss, Inc., New York, N.Y. (1990)
Huston et al., Cell Biophysics, 22:189-224 (1993)
Pellegrino et al, Bioconjugate Chem. 2020, 31, 1775-1783
Eylenstein R, Weinfurtner D, Härtle S, Strohner R, Böttcher J, Augustin M, Ostendorp R, Steidl S. Molecular basis of in vitro affinity maturation and functional evolution of a neutralizing anti-human GM-CSF antibody. MAbs. 2016;8(1):176-86
Harding FA, Stickler MM, Razo J, DuBridge RB. The immunogenicity of humanized and fully human antibodies: residual immunogenicity resides in the CDR regions. MAbs. 2010 May-Jun;2(3):256-65.
Schanzer, J.M., et al., A novel glycoengineered bispecific antibody format for targeted inhibition of epidermal growth factor receptor (EGFR) and insulin-like growth factor receptor type I (IGF-1R) demonstrating unique molecular properties. J Biol Chem, 2014. 289(27): p. 18693-706.
Goebeler, M.E. and R. Bargou, Blinatumomab: a CD19/CD3 bispecific T cell engager (BiTE) with unique anti-tumor efficacy. Leuk Lymphoma, 2016. 57(5): p. 1021-32.
Neri, D., et al., High-affinity antigen binding by chelating recombinant antibodies (CRAbs). J Mol Biol, 1995. 246(3): p. 367-73.
Nagorsen, D., et al., Blinatumomab: a historical perspective. Pharmacol Ther, 2012. 136(3): p. 334-42
Cazzamalli S. et al, Chemically Defined Antibody- and Small Molecule-Drug Conjugates for in Vivo Tumor Targeting Applications: A Comparative Analysis. J Am Chem Soc. 2018 Feb 7;140(5):1617-1621
Spiess C et al., Alternative molecular formats and therapeutic applications for bispecific antibodies.Mol Immunol. 2015 Oct;67(2 Pt A):95-106.
Wang, A systematic approach for analysis and characterization of mispairing in bispecific antibodies with asymmetric architecture. MAbs. 2018 Nov-Dec;10(8):1226-1235
Ceuppens JL et al, CD3 modulation inhibits pokeweed mitogen-induced T-cell help for immunoglobulin production. Cell Immunol. 1986 Oct 1;102(1):144-51.
Borst J et al., BMA031, a monoclonal antibody suited to identify the T-cell receptor alpha beta/CD3 complex on viable human T lymphocytes in normal and disease states. Hum Immunol. 1990 Nov;29(3):175-88.
Li B et al., Construction and characterization of a humanized anti-human CD3 monoclonal antibody 12F6 with effective immunoregulation functions. Immunology. 2005 Dec;116(4):487-98.

### Sequences

The following sequences form part of the disclosure of the present application. A WIPO ST 25 compatible electronic sequence listing is provided with this application, too. For the avoidance of doubt, if discrepancies exist between the sequences in the following table and the electronic sequence listing, the sequences in this table shall be deemed to be the correct ones.

| **No** | **Sequence** | **Type** |
|---|---|---|
| 1 | DIKLQQSGAELARPGASVKMSCKTSGYTFTRYTMHWVKQRPGQGLEWIGY INPSRGYTNYNQKFKDKATLTTDKSSSTAYMQLSSLTSEDSAVYYCARYY DDHYCLDYWGQGTTLTVSS | HCVD of anti CD3 BlinCD3 |
| 2 | YTFTRYTMH | HCDR1 of anti CD3 BlinCD3 |
| 3 | WIGYINPSRGYTNY | HCDR2 of anti CD3 BlinCD3 |
| 4 | RYYDDHYCLDY | HCDR3 of anti CD3 BlinCD3 |
| 5 | GYTFTRY | alternative HCDR1 of anti CD3 BlinCD3 |
| 6 | NPSRGY | alternative HCDR2 of anti CD3 BlinCD3 |
| 7 | YYDDHYCLDY | alternative HCDR3 of anti CD3 BlinCD3 |
| 8 | DIQLTQSPAIMSASPGEKVTMTCRASSSVSYMNWYQQKSGTSPKRWIYDT SKVASGVPYRFSGSGSGTSYSLTISSMEAEDAATYYCQQWSSNPLTFGAG TKLELK | LCVD of anti CD3 BlinCD3 |
| 9 | SSVSYMN | LCDR1 of anti CD3 BlinCD3 |
| 10 | RWIYDTSKVAS | LCDR2 of anti CD3 BlinCD3 |
| 11 | QQWSSNPL | LCDR3 of anti CD3 BlinCD3 |
| 12 | RASSSVSYMN | alternative LCDR1 of anti CD3 BlinCD3 |
| 13 | DTSKVAS | alternative LCDR2 of anti CD3 BlinCD3 |
| 14 | QQWSSNPLT | alternative LCDR3 of anti CD3 BlinCD3 |
| 15 | DIKLQQSGAELARPGASVKMSCKTSGYTFTRYTMHWVKQRPGQGLEWIGY INPSRGYTNYNQKFKDKATLTTDKSSSTAYMQLSSLTSEDSAVYYCARYY DDHYCLDYWGQGTTLTVSSVEGGSGGSGGSGGSGGVDDIQLTQSPAIMSA SPGEKVTMTCRASSSVSYMNWYQQKSGTSPKRWIYDTSKVASGVPYRFSG SGSGTSYSLTISSMEAEDAATYYCQQWSSNPLTFGAGTKLELK | scFV sequence of anti CD3 (VH-Linker-VL) BlinCD3 |
| 16 | | HCVD of anti CD3 OKT3 |
| 17 | YTFTRYTMH | HCDR1 of anti CD3 OKT3 |
| 18 | WIGYINPSRGYTNY | HCDR2 of anti CD3 OKT3 |
| 19 | RYYDDHYCLDY | HCDR3 of anti CD3 OKT3 |
| 20 | QIVLTQSPAIMSASPGEKVTMTCSASSSVSYMNWYQQKSGTSPKRWIYDT SKLASGVPAHFRGSGSGTSYSLTISGMEAEDAATYYCQQWSSNPFTFGSG TKLEINR | LCVD of anti CD3 OKT3 |
| 21 | SSVSYMN | LCDR1 of anti CD3 OKT3 |
| 22 | RWIYDTSKLAS | LCDR2 of anti CD3 OKT3 |
| 23 | QQWSSNPF | LCDR3 of anti CD3 OKT3 |
| 24 | QVQLQQSGAELARPGASVKMSCKASGYTFTRYTMHWVKQRPGQGLEWIGY INPSRGYTNYNQKFKDKATLTTDKSSSTAYMQLSSLTSEDSAVYYCARYY DDHYCLDYWGQGTTLTVSSVEGGSGGSGGSGGSGGVDQIVLTQSPAIMSA SPGEKVTMTCSASSSVSYMNWYQQKSGTSPKRWIYDTSKLASGVPAHFRG SGSGTSYSLTISGMEAEDAATYYCQQWSSNPFTFGSGTKLEINR | scFV sequence of anti CD3 (VH-Linker-VL) |
| 25 | MGWSLILLFLVAVATGVHS | Signal peptide |
| 26 | VEGGSGGSGGSGGSGGVD | Linker in BlinCD3 and Okt 3 scFv |
| 27 | | HCVD of E2 (DP47) |
| 28 | FTFGSFSMS | HCDR1 of E2 (DP47) |
| 29 | WVAGLSARSSLTHY | HCDR2 of E2 (DP47) |
| 30 | RSYDSSGYWGHFYSYMDVW | HCDR3 of (DP47) |
| 31 | QSVLTQPSSVSAAPGQKVTISCSGSTSNIGNNYVSWYQQHPGKAPKLMIY DVSKRPSGVPDRFSGSKSGNSASLDISGLQSEDEADYYCAAWDDSLSEFL FGTGTKLTVL | LCVD of E2 (DPL5/2) |
| 32 | TSNIGNNYVS | LCDR1 of E2 (DPL5/2) |
| 33 | LMIYDVSKRPS | LCDR2 of E2 (DPL5/2) |
| 34 | AAWDDSLSEFL | LCDR3 of E2 (DPL5/2) |
| 35 | QVQLVESGGNLVQPGGSLRLSCAASGFTFGSFSMSWVRQAPGGGLEWVAG LSARSSLTHYADSVKGRFTISRDNAKNSVYLQMNSLRVEDTAVYYCARRS YDSSGYWGHFYSYMDVWGQGTLVTVS GGGGSGGGGSGGGGS QSVLTQPSSVSAAPGQKVTISCSGSTSNIGNNYVSWYQQHPGKAPKLMIY DVSKRPSGVPDRFSGSKSGNSASLDISGLQSEDEADYYCAAWDDSLSEFL FGTGTKLTVL | scFV sequence of E2 (VH-Linker-VL) |
| 36 | DVVMTQTPLSLPVSLGDQASISCRSSQSLVHSNGNTYLRWYLQKPGQSPK VLIYKVSNRVSGVPDRFSGSGSGTDFTLKINRVEAEDLGVYFCSQSTHVP WTFGGGTKLEIKSS | HCVD of 4m5.3 |
| 37 | QSLVHSNGNTYLR | HCDR1 of 4m5.3 |
| 38 | LIYKVSNRVS | HCDR2 of 4m5.3 |
| 39 | SQSTHVPW | HCDR3 of 4m5.3 |
| 40 | VKLDETGGGLVQPGGAMKLSCVTSGFTFGHYWMNWVRQSPEKGLEWVAQF RNKPYNYETYYSDSVKGRFTISRDDSKSSVYLQMNNLRVEDTGIYYCTGA SYGMEYLGQGTSVTVS | LCVD of 4m5.3 |
| 41 | FTFGHYWMN | LCDR1 of 4m5.3 |
| 42 | WVAQFRNKPYNYETYY | LCDR2 of 4m5.3 |
| 43 | GASYGMEYL | LCDR3 of 4m5.3 |
| 44 | DVVMTQTPLSLPVSLGDQASISCRSSQSLVHSNGNTYLRWYLQKPGQSPK VLIYKVSNRVSGVPDRFSGSGSGTDFTLKINRVEAEDLGVYFCSQSTHVP WTFGGGTKLEIKSS ADDAKKDAAKKDDAKKDDAKKDGG VKLDETGGGLVQPGGAMKLSCVTSGFTFGHYWMNWVRQSPEKGLEWVAQF RNKPYNYETYYSDSVKGRFTISRDDSKSSVYLQMNNLRVEDTGIYYCTGA SYGMEYLGQGTSVTVS | scFV sequence of 4m5.3 (VH-Linker-VL) |
| 45 | GGGGSGGGGSGGGGS | linker in E2 |
| 46 | ADDAKKDAAKKDDAKKDDAKKDGG | Linker in 4m5.3 scFv |
| 47 | DVYHDGACPEVKPVDNFDWSQYHGKWWEVAKYPSPNGKYGKCGWIEYTPE GKSVKVSRYDVIHGKEYFMEGTAYPVGDSKIGKIYHSRTVGGYTKKTVFN VLSTDNKNYIIGYRCRYDEDKKGHWDHVWVLSRSMVLTGEAKTAVENYLI GSPVVDSQKLVYSDFSEAACKVNNSNWSHPQFEK | Anticalin FluA (R95K/A451/S114R variant) |
| 48 | QVQLVESGGNLVQPGGSLRLSCAASGFTFGSFSMSWVRQAPGGGLEWVAG LSARSSLTHYADSVKGRFTISRDNAKNSVYLQMNSLRVEDTAVYYCARRS YDSSGYWGHFYSYMDVWGQGTLVTVS GGGGSGGGGSGGGGS QSVLTQPSSVSAAPGQKVTISCSGSTSNIGNNYVSWYQQHPGKAPKLMIY DVSKRPSGVPDRFSGSKSGNSASLDISGLQSEDEADYYCAAWDDSLSEFL FGTGTKLTVL GGGGS DIKLQQSGAELARPGASVKMSCKTSGYTFTRYTMHWVKQRPGQGLEWIGY INPSRGYTNYNQKFKDKATLTTDKSSSTAYMQLSSLTSEDSAVYYCARYY DDHYCLDYWGQGTTLTVSS VEGGSGGSGGSGGSGGVD DIQLTQSPAIMSASPGEKVTMTCRASSSVSYMNWYQQKSGTSPKRWIYDT SKVASGVPYRFSGSGSGTSYSLTISSMEAEDAATYYCQQWSSNPLTFGAG TKLELK HHHHHH | Human VH (DP47)-(GGGGS)3 linker-Human VL (DPL5/2)-(GGGGS) linker-OKT3opt VH -linker-OKT3opt VL 6xHis tag |
| 49 | DVVMTQTPLSLPVSLGDQASISCRSSQSLVHSNGNTYLRWYLQKPGQSPK VLIYKVSNRVSGVPDRFSGSGSGTDFTLKINRVEAEDLGVYFCSQSTHVP WTFGGGTKLEIKSS ADDAKKDAAKKDDAKKDDAKKDGG VKLDETGGGLVQPGGAMKLSCVTSGFTFGHYWMNWVRQSPEKGLEWVAQF RNKPYNYETYYSDSVKGRFTISRDDSKSSVYLQMNNLRVEDTGIYYCTGA SYGMEYLGQGTSVTVS GGGGS DIKLQQSGAELARPGASVKMSCKTSGYTFTRYTMHWVKQRPGQGLEWIGY INPSRGYTNYNQKFKDKATLTTDKSSSTAYMQLSSLTSEDSAVYYCARYY DDHYCLDYWGQGTTLTVSS VEGGSGGSGGSGGSGGVD DIQLTQSPAIMSASPGEKVTMTCRASSSVSYMNWYQQKSGTSPKRWIYDT SKVASGVPYRFSGSGSGTSYSLTISSMEAEDAATYYCQQWSSNPLTFGAG | Murine VL (4m5.3) - original linker - Murine VH (4m5.3)- (GGGGS) linker - OKT3opt VH - linker - OKT3opt VL 6xHis tag |
| | TKLELK HHHHHH | |
| 50 | DVYHDGACPEVKPVDNFDWSQYHGKWWEVAKYPSPNGKYGKCGWIEYTPE GKSVKVSRYDVIHGKEYFMEGTAYPVGDSKIGKIYHSRTVGGYTKKTVFN VLSTDNKNYIIGYRCRYDEDKKGHWDHVWVLSRSMVLTGEAKTAVENYLI GSPVVDSQKLVYSDFSEAACKVNNSNWSHPQFEK GGGGS DIKLQQSGAELARPGASVKMSCKTSGYTFTRYTMHWVKQRPGQGLEWIGY INPSRGYTNYNQKFKDKATLTTDKSSSTAYMQLSSLTSEDSAVYYCARYY DDHYCLDYWGQGTTLTVSS VEGGSGGSGGSGGSGGVD DIQLTQSPAIMSASPGEKVTMTCRASSSVSYMNWYQQKSGTSPKRWIYDT SKVASGVPYRFSGSGSGTSYSLTISSMEAEDAATYYCQQWSSNPLTFGAG TKLELK HHHHHH | FluA- (GGGGS) linker-OKT3opt VH - linker-OKT3opt VL 6xHis tag |
| 51 | DIKLQQSGAELARPGASVKMSCKTSGYTFTRYTMHWVKQRPGQGLEWIGY INPSRGYTNYNQKFKDKATLTTDKSSSTAYMQLSSLTSEDSAVYYCARYY DDHYCLDYWGQGTTLTVSS VEGGSGGSGGSGGSGGVD DIQLTQSPAIMSASPGEKVTMTCRASSSVSYMNWYQQKSGTSPKRWIYDT SKVASGVPYRFSGSGSGTSYSLTISSMEAEDAATYYCQQWSSNPLTFGAG TKLELK AAA EQKLISEEDLGS HHHHHH C | OKT3opt VH - linker-OKT3opt VL - Ala₃ - Myc tag - 6xHis tag-Cysteine |
| 52 | SEVQLVESGGGLVQPGGSLRLSCAASGFNISVYMMHWVRQAPGKGLEWVA SIYPYSGYTYYADSVKGRFTISADTSKNTAYLQMNSLRAEDTAVYYCARY VYHALDYWGQGTLVTVSS | HCVD of anti CD117 79D |
| 53 | FNISVYMMH | HCDR1 of anti CD117 79D |
| 54 | WVASIYPYSGYTYY | HCDR2 of anti CD117 79D |
| 55 | RYVYHALDY | HCDR3 of anti CD117 79D |
| 56 | GFNISVY | alternative HCDR1 of anti CD117 79D |
| 57 | YPYSGY | alternative HCDR2 of anti CD117 79D |
| 58 | YVYHALDY | alternative HCDR3 of anti CD117 79D |
| 59 | DIQMTQSPSSLSASVGDRVTITCRASQRGLRNVAVAWYQQKPGKAPKLLI YSASSLYSGVPSRFSGSRSGTDFTLTISSLQPEDFATYYCQQWAVHSLIT FGQGTKVEIKR | LCVD of anti CD117 79D |
| 60 | QRGLRNVAVA | LCDR1 of anti CD117 79D |
| 61 | LLIYSASSLYS | LCDR2 of anti CD117 79D |
| 62 | QQWAVHSLI | LCDR3 of anti CD117 79D |
| 63 | RASQRGLRNVAVA | alternative LCDR1 of anti CD117 79D |
| 64 | SASSLYS | alternative LCDR2 of anti CD117 79D |
| 65 | QQWAVHSLIT | alternative LCDR3 of anti CD117 79D |
| 66 | DIQMTQSPSSLSASVGDRVTITCRASQRGLRNVAVAWYQQKPGKAPKLLI YSASSLYSGVPSRFSGSRSGTDFTLTISSLQPEDFATYYCQQWAVHSLIT FGQGTKVEIKRGGGGSGGGGSGGGGSEVQLVESGGGLVQPGGSLRLSCAA SGFNISVYMMHWVRQAPGKGLEWVASIYPYSGYTYYADSVKGRFTISADT SKNTAYLQMNSLRAEDTAVYYCARYVYHALDYWGQGTLVTVSS | scFV sequence of anti CD117 (VL-Linker-VH) |
| 67 | GGGGS | linker |
| 68 | EVQLLESGGGLVQPGGSLRLSCAASGFTFSSYAMSWVRQAPGKGLEWVSA IDGSGGSTYYADSVKGRFTISRDNSKNTLYLQMNSLRAEDTAVYYCVKGP PVFDYWGQGTLVTVSS | HCVD of anti CAIX (XE114) |
| 69 | FTFSSYAMS | HCDR1 of anti CAIX XE114 |
| 70 | WVSAIDGSGGSTYYA | HCDR2 of anti CAIX XE114 |
| 71 | VKGPPVFDY | HCDR3 of anti CAIX XE114 |
| 72 | SSELTQDPAVSVALGQTVRITCQGDSLRSYYASWYQQKPGQAPVLVIYGK NNRPSGIPDRFSGSSSGNTASLTITGAQAEDEADYYCQSSKWSWDPVVFG GGTKLTVL | HCVD of anti CAIX (XE114) |
| 73 | SLRSYYAS | LCDR1 of anti CAIX XE114 |
| 74 | LVIYGKNNRPS | LCDR2 of anti CAIX XE114 |
| 75 | QSSKWSWDPV | LCDR3 of anti CAIX XE114 |

## Claims

1. A bispecific binding agent comprising at least a first binding domain and a second binding domain, wherein said first binding domain binds to an organic fluorophore and wherein said second binding domain binds to CD3.

2. The bispecific binding agent according to any one of the aforementioned claims, wherein said first binding domain binds to Fluorescein, preferably Fluorescein when bound to a protein ("conjugated Fluorescein").

3. The bispecific binding agent according to any one of the aforementioned claims, wherein the first binding domain which binds to Fluorescein or conjugated Fluorescein
a) comprises a set of heavy chain/light chain complementarity determining regions (CDR) comprised in the heavy chain/light variable region sequence pair of one of the anti Fluorescein antibodies E2 or 4m5.3, as set forth in SEQ ID NOs 27 and 31, or SEQ ID NOs 36 and 40, respectively
b) comprises the set of heavy chain/light chain complementarity determining regions (CDR) comprising the following sequences
• HC CDR1 (SEQ ID NO 28)
• HC CDR2 (SEQ ID NO 29)
• HC CDR3 (SEQ ID NO 30)
• LC CDR1 (SEQ ID NO 32)
• LC CDR2 (SEQ ID NO 33), and
• LC CDR3 (SEQ ID NO 34)
c) comprises the set of heavy chain/light chain complementarity determining regions (CDR) comprising the following sequences
• HC CDR1 (SEQ ID NO 37)
• HC CDR2 (SEQ ID NO 38)
• HC CDR3 (SEQ ID NO 39)
• LC CDR1 (SEQ ID NO 41)
• LC CDR2 (SEQ ID NO 42), and
• LC CDR3 (SEQ ID NO 43)
d) comprises the heavy chain/light chain complementarity determining regions (CDR) of b) or c), with the proviso that at least one of the CDRs has up to 3 amino acid substitutions relative to the respective SEQ ID NO 28 - 30, 32 - 34, 37 - 39 or 41 - 43, and/or
e) comprises the heavy chain/light chain complementarity determining regions (CDR) of b), with the proviso that at least one of the CDRs has a sequence identity of ≥ 66 % to the respective SEQ ID NO 28 - 30, 32 - 34, 37 - 39 or 41 - 43,
wherein the CDRs are embedded in a suitable protein framework so as to be capable to bind to Fluorescein or conjugated Fluorescein.

4. The bispecific binding agent according to any one of the aforementioned claims, wherein the first binding domain which binds to Fluorescein or conjugated Fluorescein comprises
a) the variable domains of one antibody selected from the group consisting of E2 or 4m5.3
b) the heavy chain/light chain variable domains (VD)
• HC VD (SEQ ID NO 27 or 36), and
• LC VD (SEQ ID NO 31 or 40)
c) the heavy chain/light chain variable domains (VD) of b), with the proviso that
• the HCVD has a sequence identity of ≥ 80 % to the respective SEQ ID NO 27 or 36, and/or
d) the LCVD has a sequence identity of ≥ 80 % to the respective SEQ ID NO 31 or 40the heavy chain/light chain variable domains (VD) of b), with the proviso that at least one of the HCVD or LCVD has up to 10 amino acid substitutions relative to the respective SEQ ID NOs,
wherein said bispecific binding agent is still capable to bind to Fluorescein or conjugated Fluorescein.

5. The bispecific binding agent according to any one of the aforementioned claims, wherein the second binding domain which binds to CD3
a) comprises a set of heavy chain/light chain complementarity determining regions (CDR) comprised in the heavy chain/light variable region sequence pair of one of the anti CD3 antibodies BlinCD3 and OKT3, as set forth in SEQ ID NOs 1 and 8, or SEQ ID NOs 16 and 20, respectively
b) comprises the set of heavy chain/light chain complementarity determining regions (CDR) comprising the following sequences
• HC CDR1 (SEQ ID NO 2 or 5)
• HC CDR2 (SEQ ID NO 3 or 6)
• HC CDR3 (SEQ ID NO 4 or 7)
• LC CDR1 (SEQ ID NO 9 or 12)
• LC CDR2 (SEQ ID NO 10 or 13), and
• LC CDR3 (SEQ ID NO 11 or 14)
c) comprises the set of heavy chain/light chain complementarity determining regions (CDR) comprising the following sequences
• HC CDR1 (SEQ ID NO 17)
• HC CDR2 (SEQ ID NO 18)
• HC CDR3 (SEQ ID NO 19)
• LC CDR1 (SEQ ID NO 21)
• LC CDR2 (SEQ ID NO 22), and
• LC CDR3 (SEQ ID NO 23)
d) comprises the heavy chain/light chain complementarity determining regions (CDR) of b) or c), with the proviso that at least one of the CDRs has up to 3 amino acid substitutions relative to the respective SEQ ID NO 2 - 4, 9 - 11, 17 - 19 or 21 - 23, and/or
e) comprises the heavy chain/light chain complementarity determining regions (CDR) of b), with the proviso that at least one of the CDRs has a sequence identity of ≥ 66 % to the respective SEQ ID NO 2 - 4, 9 - 11, 17 - 19 or 21 - 23,
wherein the CDRs are embedded in a suitable protein framework so as to be capable to bind to CD3.

6. The bispecific binding agent according to any one of the aforementioned claims, wherein the second binding domain which binds to CD3 comprises
a) the variable domains of one antibody selected from the group consisting of OKT3 and BlinCD3
b) the heavy chain/light chain variable domains (VD)
• HC VD (SEQ ID NO 1 or 16), and
• LC VD (SEQ ID NO 8 or 20)
c) the heavy chain/light chain variable domains (VD) of b), with the proviso that
• the HCVD has a sequence identity of ≥ 80 % to the respective SEQ ID NO 1 or 16, and/or
d) the LCVD has a sequence identity of ≥ 80 % to the respective SEQ ID NO 8 or 20the heavy chain/light chain variable domains (VD) of b), with the proviso that at least one of the HCVD or LCVD has up to 10 amino acid substitutions relative to the respective SEQ ID NOs,
wherein said bispecific binding agent is still capable to bind to CD3.

7. The bispecific binding agent according to any one of the aforementioned claims, wherein the second binding domain which binds to CD3
a) comprises a scFv-like sequence as set forth in any one of SEQ ID NOs 15 or 24, or
b) comprises an amino acid sequence that has a sequence identity of ≥ 80 % to any one of SEQ ID NOs 15 or 24.

8. The bispecific binding agent according to any one of the aforementioned claims, wherein the first binding domain which binds to Fluorescein or conjugated Fluorescein
c) comprises a scFv-like sequence as set forth in any one of SEQ ID NOs 35 or 44, or
d) comprises an amino acid sequence that has a sequence identity of ≥ 80 % to any one of SEQ ID NOs 35 or 44.

9. The bispecific binding agent according to any one of the aforementioned claims, which
a) comprises at least one of the sequences selected from SEQ ID NOs 48 - 50, or
b) comprises an amino acid sequence that has a sequence identity of ≥ 80 % to SEQ ID NOs 48 - 50,
with optionally a C terminal cysteine residue and/or a His tag removed or in place.

10. A combination comprising
a) the bispecific binding agent according to any one of claims 1 - 9 and
b) a labelled binding agent that binds specifically to a target antigen,
wherein the labelled binding agent is labelled with an organic fluorophore that is specifically detected by the first binding domain of the binding agent according to any one of claims 1 - 9.

11. A dosage regimen, comprising administration to a patient, consecutively or concomitantly of
a) the bispecific binding agent according to any one of claims 1 - 9 and
b) a labelled binding agent that binds specifically to a target antigen, and is labelled with an organic fluorophore that is specifically detected by the first binding domain of the binding agent according to any one of claims 1 - 9.

12. The combination or dosage regimen according to claim 10 or 11, wherein the organic fluorophore in the labelled binding agent is fluorescein.

13. The combination or dosage regimen according to any one of claims 10 - 12, wherein the labelled binding agent comprises an antibody, or a target binding fragment or derivative thereof.

14. A pharmaceutical composition comprising the combination according to any one of claims 10, 12 or 13 and optionally one or more pharmaceutically acceptable excipients.

15. The combination, dosage regimen or pharmaceutical composition according to any one of claims 10 - 14 for use (in the manufacture of a medicament) in the treatment of a human or animal subject
• being diagnosed for,
• suffering from or
• being at risk of
developing a neoplastic disease, or for the prevention of such condition.


SEQUENCE LISTING

<110>  ETH Zürich, Universität Zürich

<120>  Bispecific binding agent that binds to CD3 and a fluorophore

<130>  2020-127

<160>  75

<170>  PatentIn version 3.5

<210>  1
<211>  119
<212>  PRT
<213>  artificial sequence

<220>
<223>  antibody sequence

<400>  1

Asp Ile Lys Leu Gln Gln Ser Gly Ala Glu Leu Ala Arg Pro Gly Ala
1               5                   10                  15

Ser Val Lys Met Ser Cys Lys Thr Ser Gly Tyr Thr Phe Thr Arg Tyr
            20                  25                  30

Thr Met His Trp Val Lys Gln Arg Pro Gly Gln Gly Leu Glu Trp Ile
        35                  40                  45

Gly Tyr Ile Asn Pro Ser Arg Gly Tyr Thr Asn Tyr Asn Gln Lys Phe
    50                  55                  60

Lys Asp Lys Ala Thr Leu Thr Thr Asp Lys Ser Ser Ser Thr Ala Tyr
65                  70                  75                  80

Met Gln Leu Ser Ser Leu Thr Ser Glu Asp Ser Ala Val Tyr Tyr Cys
                85                  90                  95

Ala Arg Tyr Tyr Asp Asp His Tyr Cys Leu Asp Tyr Trp Gly Gln Gly
            100                 105                 110

Thr Thr Leu Thr Val Ser Ser
        115

<210>  2
<211>  9
<212>  PRT
<213>  artificial sequence

<220>
<223>  antibody sequence

<400>  2

Tyr Thr Phe Thr Arg Tyr Thr Met His
1               5

<210>  3
<211>  14
<212>  PRT
<213>  artificial sequence

<220>
<223>  antibody sequence

<400>  3

Trp Ile Gly Tyr Ile Asn Pro Ser Arg Gly Tyr Thr Asn Tyr
1               5                   10

<210>  4
<211>  11
<212>  PRT
<213>  artificial sequence

<220>
<223>  antibody sequence

<400>  4

Arg Tyr Tyr Asp Asp His Tyr Cys Leu Asp Tyr
1               5                   10

<210>  5
<211>  7
<212>  PRT
<213>  artificial sequence

<220>
<223>  antibody sequence

<400>  5

Gly Tyr Thr Phe Thr Arg Tyr
1               5

<210>  6
<211>  6
<212>  PRT
<213>  artificial sequence

<220>
<223>  antibody sequence

<400>  6

Asn Pro Ser Arg Gly Tyr
1               5

<210>  7
<211>  10

<212>  PRT
<213>  artificial sequence

<220>
<223>  antibody sequence

<400>  7

Tyr Tyr Asp Asp His Tyr Cys Leu Asp Tyr
1               5                   10


<210>  8
<211>  106
<212>  PRT
<213>  artificial sequence

<220>
<223>  antibody sequence

<400>  8

Asp Ile Gln Leu Thr Gln Ser Pro Ala Ile Met Ser Ala Ser Pro Gly
1               5                   10                  15


Glu Lys Val Thr Met Thr Cys Arg Ala Ser Ser Ser Val Ser Tyr Met
            20                  25                  30


Asn Trp Tyr Gln Gln Lys Ser Gly Thr Ser Pro Lys Arg Trp Ile Tyr
        35                  40                  45


Asp Thr Ser Lys Val Ala Ser Gly Val Pro Tyr Arg Phe Ser Gly Ser
    50                  55                  60


Gly Ser Gly Thr Ser Tyr Ser Leu Thr Ile Ser Ser Met Glu Ala Glu
65                  70                  75                  80


Asp Ala Ala Thr Tyr Tyr Cys Gln Gln Trp Ser Ser Asn Pro Leu Thr
                85                  90                  95


Phe Gly Ala Gly Thr Lys Leu Glu Leu Lys
            100                 105


<210>  9
<211>  7
<212>  PRT
<213>  artificial sequence

<220>
<223>  antibody sequence

<400>  9

Ser Ser Val Ser Tyr Met Asn
1               5

<210>  10
<211>  11
<212>  PRT
<213>  artificial sequence

<220>
<223>  antibody sequence

<400>  10

Arg Trp Ile Tyr Asp Thr Ser Lys Val Ala Ser
1               5                   10


<210>  11
<211>  8
<212>  PRT
<213>  artificial sequence

<220>
<223>  antibody sequence

<400>  11

Gln Gln Trp Ser Ser Asn Pro Leu
1               5


<210>  12
<211>  10
<212>  PRT
<213>  artificial sequence

<220>
<223>  antibody sequence

<400>  12

Arg Ala Ser Ser Ser Val Ser Tyr Met Asn
1               5                   10


<210>  13
<211>  7
<212>  PRT
<213>  artificial sequence

<220>
<223>  antibody sequence

<400>  13

Asp Thr Ser Lys Val Ala Ser
1               5


<210>  14
<211>  9
<212>  PRT
<213>  artificial sequence

<220>

<223>  antibody sequence

<400>  14

Gln Gln Trp Ser Ser Asn Pro Leu Thr
1               5

<210>  15
<211>  243
<212>  PRT
<213>  artificial sequence

<220>
<223>  antibody sequence

<400>  15

Asp Ile Lys Leu Gln Gln Ser Gly Ala Glu Leu Ala Arg Pro Gly Ala
1               5                   10                  15

Ser Val Lys Met Ser Cys Lys Thr Ser Gly Tyr Thr Phe Thr Arg Tyr
            20                  25                  30

Thr Met His Trp Val Lys Gln Arg Pro Gly Gln Gly Leu Glu Trp Ile
        35                  40                  45

Gly Tyr Ile Asn Pro Ser Arg Gly Tyr Thr Asn Tyr Asn Gln Lys Phe
    50                  55                  60

Lys Asp Lys Ala Thr Leu Thr Thr Asp Lys Ser Ser Ser Thr Ala Tyr
65                  70                  75                  80

Met Gln Leu Ser Ser Leu Thr Ser Glu Asp Ser Ala Val Tyr Tyr Cys
                85                  90                  95

Ala Arg Tyr Tyr Asp Asp His Tyr Cys Leu Asp Tyr Trp Gly Gln Gly
            100                 105                 110

Thr Thr Leu Thr Val Ser Ser Val Glu Gly Gly Ser Gly Gly Ser Gly
        115                 120                 125

Gly Ser Gly Gly Ser Gly Gly Val Asp Asp Ile Gln Leu Thr Gln Ser
    130                 135                 140

Pro Ala Ile Met Ser Ala Ser Pro Gly Glu Lys Val Thr Met Thr Cys
145                 150                 155                 160

Arg Ala Ser Ser Ser Val Ser Tyr Met Asn Trp Tyr Gln Gln Lys Ser
                165                 170                 175

Gly Thr Ser Pro Lys Arg Trp Ile Tyr Asp Thr Ser Lys Val Ala Ser

180                 185                 190

Gly Val Pro Tyr Arg Phe Ser Gly Ser Gly Ser Gly Thr Ser Tyr Ser
        195                 200                 205

Leu Thr Ile Ser Ser Met Glu Ala Glu Asp Ala Ala Thr Tyr Tyr Cys
    210                 215                 220

Gln Gln Trp Ser Ser Asn Pro Leu Thr Phe Gly Ala Gly Thr Lys Leu
225                 230                 235                 240

Glu Leu Lys

<210>  16
<211>  119
<212>  PRT
<213>  artificial sequence

<220>
<223>  antibody sequence

<400>  16

<210>  17
<211>  9

<212>  PRT
<213>  artificial sequence

<220>
<223>  antibody sequence

<400>  17

Tyr Thr Phe Thr Arg Tyr Thr Met His
1               5


<210>  18
<211>  14
<212>  PRT
<213>  artificial sequence

<220>
<223>  antibody sequence

<400>  18

Trp Ile Gly Tyr Ile Asn Pro Ser Arg Gly Tyr Thr Asn Tyr
1               5                   10


<210>  19
<211>  11
<212>  PRT
<213>  artificial sequence

<220>
<223>  antibody sequence

<400>  19

Arg Tyr Tyr Asp Asp His Tyr Cys Leu Asp Tyr
1               5                   10


<210>  20
<211>  107
<212>  PRT
<213>  artificial sequence

<220>
<223>  antibody sequence

<400>  20

Gln Ile Val Leu Thr Gln Ser Pro Ala Ile Met Ser Ala Ser Pro Gly
1               5                   10                  15


Glu Lys Val Thr Met Thr Cys Ser Ala Ser Ser Ser Val Ser Tyr Met
            20                  25                  30


Asn Trp Tyr Gln Gln Lys Ser Gly Thr Ser Pro Lys Arg Trp Ile Tyr
        35                  40                  45


Asp Thr Ser Lys Leu Ala Ser Gly Val Pro Ala His Phe Arg Gly Ser

50                  55                  60

Gly Ser Gly Thr Ser Tyr Ser Leu Thr Ile Ser Gly Met Glu Ala Glu
65                  70                  75                  80

Asp Ala Ala Thr Tyr Tyr Cys Gln Gln Trp Ser Ser Asn Pro Phe Thr
                85                  90                  95

Phe Gly Ser Gly Thr Lys Leu Glu Ile Asn Arg
            100                 105

<210>  21
<211>  7
<212>  PRT
<213>  artificial sequence

<220>
<223>  antibody sequence

<400>  21

Ser Ser Val Ser Tyr Met Asn
1               5

<210>  22
<211>  11
<212>  PRT
<213>  artificial sequence

<220>
<223>  antibody sequence

<400>  22

Arg Trp Ile Tyr Asp Thr Ser Lys Leu Ala Ser
1               5                   10

<210>  23
<211>  8
<212>  PRT
<213>  artificial sequence

<220>
<223>  antibody sequence

<400>  23

Gln Gln Trp Ser Ser Asn Pro Phe
1               5

<210>  24
<211>  244
<212>  PRT
<213>  artificial sequence

<220>

<223>  antibody sequence

<400>  24

Gln Val Gln Leu Gln Gln Ser Gly Ala Glu Leu Ala Arg Pro Gly Ala
1               5                   10                  15

Ser Val Lys Met Ser Cys Lys Ala Ser Gly Tyr Thr Phe Thr Arg Tyr
            20                  25                  30

Thr Met His Trp Val Lys Gln Arg Pro Gly Gln Gly Leu Glu Trp Ile
        35                  40                  45

Gly Tyr Ile Asn Pro Ser Arg Gly Tyr Thr Asn Tyr Asn Gln Lys Phe
    50                  55                  60

Lys Asp Lys Ala Thr Leu Thr Thr Asp Lys Ser Ser Ser Thr Ala Tyr
65                  70                  75                  80

Met Gln Leu Ser Ser Leu Thr Ser Glu Asp Ser Ala Val Tyr Tyr Cys
                85                  90                  95

Ala Arg Tyr Tyr Asp Asp His Tyr Cys Leu Asp Tyr Trp Gly Gln Gly
            100                 105                 110

Thr Thr Leu Thr Val Ser Ser Val Glu Gly Gly Ser Gly Gly Ser Gly
        115                 120                 125

Gly Ser Gly Gly Ser Gly Gly Val Asp Gln Ile Val Leu Thr Gln Ser
    130                 135                 140

Pro Ala Ile Met Ser Ala Ser Pro Gly Glu Lys Val Thr Met Thr Cys
145                 150                 155                 160

Ser Ala Ser Ser Ser Val Ser Tyr Met Asn Trp Tyr Gln Gln Lys Ser
                165                 170                 175

Gly Thr Ser Pro Lys Arg Trp Ile Tyr Asp Thr Ser Lys Leu Ala Ser
            180                 185                 190

Gly Val Pro Ala His Phe Arg Gly Ser Gly Ser Gly Thr Ser Tyr Ser
        195                 200                 205

Leu Thr Ile Ser Gly Met Glu Ala Glu Asp Ala Ala Thr Tyr Tyr Cys
    210                 215                 220

Gln Gln Trp Ser Ser Asn Pro Phe Thr Phe Gly Ser Gly Thr Lys Leu
225                 230                 235                 240

Glu Ile Asn Arg

<210> 25
<211> 19
<212> PRT
<213> artificial sequence

<220>
<223> antibody sequence

<400> 25

Met Gly Trp Ser Leu Ile Leu Leu Phe Leu Val Ala Val Ala Thr Gly
1               5                   10                  15

Val His Ser

<210> 26
<211> 18
<212> PRT
<213> artificial sequence

<220>
<223> antibody sequence

<400> 26

Val Glu Gly Gly Ser Gly Gly Ser Gly Gly Ser Gly Gly Ser Gly Gly
1               5                   10                  15

Val Asp

<210> 27
<211> 126
<212> PRT
<213> artificial sequence

<220>
<223> antibody sequence

<400> 27

<210>  28
<211>  9
<212>  PRT
<213>  artificial sequence

<220>
<223>  antibody sequence

<400>  28

Phe Thr Phe Gly Ser Phe Ser Met Ser
1               5

<210>  29
<211>  14
<212>  PRT
<213>  artificial sequence

<220>
<223>  antibody sequence

<400>  29

Trp Val Ala Gly Leu Ser Ala Arg Ser Ser Leu Thr His Tyr
1               5                   10

<210>  30
<211>  19
<212>  PRT
<213>  artificial sequence

<220>
<223>  antibody sequence

<400>  30

Arg Ser Tyr Asp Ser Ser Gly Tyr Trp Gly His Phe Tyr Ser Tyr Met
1               5                   10                  15

Asp Val Trp

<210>  31
<211>  110
<212>  PRT
<213>  artificial sequence

<220>
<223>  antibody sequence

<400>  31

Gln Ser Val Leu Thr Gln Pro Ser Ser Val Ser Ala Ala Pro Gly Gln
1               5                   10                  15

Lys Val Thr Ile Ser Cys Ser Gly Ser Thr Ser Asn Ile Gly Asn Asn
            20                  25                  30

Tyr Val Ser Trp Tyr Gln Gln His Pro Gly Lys Ala Pro Lys Leu Met
        35                  40                  45

Ile Tyr Asp Val Ser Lys Arg Pro Ser Gly Val Pro Asp Arg Phe Ser
    50                  55                  60

Gly Ser Lys Ser Gly Asn Ser Ala Ser Leu Asp Ile Ser Gly Leu Gln
65                  70                  75                  80

Ser Glu Asp Glu Ala Asp Tyr Tyr Cys Ala Ala Trp Asp Asp Ser Leu
                85                  90                  95

Ser Glu Phe Leu Phe Gly Thr Gly Thr Lys Leu Thr Val Leu
            100                 105                 110


<210>  32
<211>  10
<212>  PRT
<213>  artificial sequence

<220>
<223>  antibody sequence

<400>  32

Thr Ser Asn Ile Gly Asn Asn Tyr Val Ser
1               5                   10


<210>  33
<211>  11
<212>  PRT
<213>  artificial sequence

<220>
<223>  antibody sequence

<400>  33

Leu Met Ile Tyr Asp Val Ser Lys Arg Pro Ser
1               5                   10


<210>  34
<211>  11
<212>  PRT
<213>  artificial sequence

<220>
<223>  antibody sequence

<400>  34

Ala Ala Trp Asp Asp Ser Leu Ser Glu Phe Leu
1               5                   10


<210>  35
<211>  251
<212>  PRT
<213>  artificial sequence

<220>
<223>  antibody sequence

<400>  35

Gln Val Gln Leu Val Glu Ser Gly Gly Asn Leu Val Gln Pro Gly Gly
1               5                   10                  15


Ser Leu Arg Leu Ser Cys Ala Ala Ser Gly Phe Thr Phe Gly Ser Phe
            20                  25                  30


Ser Met Ser Trp Val Arg Gln Ala Pro Gly Gly Gly Leu Glu Trp Val
        35                  40                  45


Ala Gly Leu Ser Ala Arg Ser Ser Leu Thr His Tyr Ala Asp Ser Val
    50                  55                  60


Lys Gly Arg Phe Thr Ile Ser Arg Asp Asn Ala Lys Asn Ser Val Tyr
65                  70                  75                  80


Leu Gln Met Asn Ser Leu Arg Val Glu Asp Thr Ala Val Tyr Tyr Cys
                85                  90                  95


Ala Arg Arg Ser Tyr Asp Ser Ser Gly Tyr Trp Gly His Phe Tyr Ser
            100                 105                 110


Tyr Met Asp Val Trp Gly Gln Gly Thr Leu Val Thr Val Ser Gly Gly
        115                 120                 125


Gly Gly Ser Gly Gly Gly Gly Ser Gly Gly Gly Gly Ser Gln Ser Val

130                 135                 140

Leu Thr Gln Pro Ser Ser Val Ser Ala Ala Pro Gly Gln Lys Val Thr
145                 150                 155                 160

Ile Ser Cys Ser Gly Ser Thr Ser Asn Ile Gly Asn Asn Tyr Val Ser
                165                 170                 175

Trp Tyr Gln Gln His Pro Gly Lys Ala Pro Lys Leu Met Ile Tyr Asp
            180                 185                 190

Val Ser Lys Arg Pro Ser Gly Val Pro Asp Arg Phe Ser Gly Ser Lys
        195                 200                 205

Ser Gly Asn Ser Ala Ser Leu Asp Ile Ser Gly Leu Gln Ser Glu Asp
    210                 215                 220

Glu Ala Asp Tyr Tyr Cys Ala Ala Trp Asp Asp Ser Leu Ser Glu Phe
225                 230                 235                 240

Leu Phe Gly Thr Gly Thr Lys Leu Thr Val Leu
                245                 250

<210>  36
<211>  114
<212>  PRT
<213>  artificial sequence

<220>
<223>  antibody sequence

<400>  36

Asp Val Val Met Thr Gln Thr Pro Leu Ser Leu Pro Val Ser Leu Gly
1               5                   10                  15

Asp Gln Ala Ser Ile Ser Cys Arg Ser Ser Gln Ser Leu Val His Ser
            20                  25                  30

Asn Gly Asn Thr Tyr Leu Arg Trp Tyr Leu Gln Lys Pro Gly Gln Ser
        35                  40                  45

Pro Lys Val Leu Ile Tyr Lys Val Ser Asn Arg Val Ser Gly Val Pro
    50                  55                  60

Asp Arg Phe Ser Gly Ser Gly Ser Gly Thr Asp Phe Thr Leu Lys Ile
65                  70                  75                  80

Asn Arg Val Glu Ala Glu Asp Leu Gly Val Tyr Phe Cys Ser Gln Ser
                85                  90                  95

Thr His Val Pro Trp Thr Phe Gly Gly Gly Thr Lys Leu Glu Ile Lys
            100                 105                 110

Ser Ser

<210>  37
<211>  13
<212>  PRT
<213>  artificial sequence

<220>
<223>  antibody sequence

<400>  37

Gln Ser Leu Val His Ser Asn Gly Asn Thr Tyr Leu Arg
1               5                   10

<210>  38
<211>  10
<212>  PRT
<213>  artificial sequence

<220>
<223>  antibody sequence

<400>  38

Leu Ile Tyr Lys Val Ser Asn Arg Val Ser
1               5                   10

<210>  39
<211>  8
<212>  PRT
<213>  artificial sequence

<220>
<223>  antibody sequence

<400>  39

Ser Gln Ser Thr His Val Pro Trp
1               5

<210>  40
<211>  116
<212>  PRT
<213>  artificial sequence

<220>
<223>  antibody sequence

<400>  40

Val Lys Leu Asp Glu Thr Gly Gly Gly Leu Val Gln Pro Gly Gly Ala

1               5                   10                  15

Met Lys Leu Ser Cys Val Thr Ser Gly Phe Thr Phe Gly His Tyr Trp
            20                  25                  30

Met Asn Trp Val Arg Gln Ser Pro Glu Lys Gly Leu Glu Trp Val Ala
        35                  40                  45

Gln Phe Arg Asn Lys Pro Tyr Asn Tyr Glu Thr Tyr Tyr Ser Asp Ser
    50                  55                  60

Val Lys Gly Arg Phe Thr Ile Ser Arg Asp Asp Ser Lys Ser Ser Val
65                  70                  75                  80

Tyr Leu Gln Met Asn Asn Leu Arg Val Glu Asp Thr Gly Ile Tyr Tyr
                85                  90                  95

Cys Thr Gly Ala Ser Tyr Gly Met Glu Tyr Leu Gly Gln Gly Thr Ser
            100                 105                 110

Val Thr Val Ser
        115

<210>  41
<211>  9
<212>  PRT
<213>  artificial sequence

<220>
<223>  antibody sequence

<400>  41

Phe Thr Phe Gly His Tyr Trp Met Asn
1               5

<210>  42
<211>  16
<212>  PRT
<213>  artificial sequence

<220>
<223>  antibody sequence

<400>  42

Trp Val Ala Gln Phe Arg Asn Lys Pro Tyr Asn Tyr Glu Thr Tyr Tyr
1               5                   10                  15

<210>  43
<211>  9
<212>  PRT
<213>  artificial sequence

<220>
<223>  antibody sequence

<400>  43

Gly Ala Ser Tyr Gly Met Glu Tyr Leu
1               5


<210>  44
<211>  254
<212>  PRT
<213>  artificial sequence

<220>
<223>  antibody sequence

<400>  44

Asp Val Val Met Thr Gln Thr Pro Leu Ser Leu Pro Val Ser Leu Gly
1               5                   10                  15

Asp Gln Ala Ser Ile Ser Cys Arg Ser Ser Gln Ser Leu Val His Ser
            20                  25                  30

Asn Gly Asn Thr Tyr Leu Arg Trp Tyr Leu Gln Lys Pro Gly Gln Ser
        35                  40                  45

Pro Lys Val Leu Ile Tyr Lys Val Ser Asn Arg Val Ser Gly Val Pro
    50                  55                  60

Asp Arg Phe Ser Gly Ser Gly Ser Gly Thr Asp Phe Thr Leu Lys Ile
65                  70                  75                  80

Asn Arg Val Glu Ala Glu Asp Leu Gly Val Tyr Phe Cys Ser Gln Ser
                85                  90                  95

Thr His Val Pro Trp Thr Phe Gly Gly Gly Thr Lys Leu Glu Ile Lys
            100                 105                 110

Ser Ser Ala Asp Asp Ala Lys Lys Asp Ala Ala Lys Lys Asp Asp Ala
        115                 120                 125

Lys Lys Asp Asp Ala Lys Lys Asp Gly Gly Val Lys Leu Asp Glu Thr
    130                 135                 140

Gly Gly Gly Leu Val Gln Pro Gly Gly Ala Met Lys Leu Ser Cys Val
145                 150                 155                 160

Thr Ser Gly Phe Thr Phe Gly His Tyr Trp Met Asn Trp Val Arg Gln
                165                 170                 175

Ser Pro Glu Lys Gly Leu Glu Trp Val Ala Gln Phe Arg Asn Lys Pro
            180                 185                 190

Tyr Asn Tyr Glu Thr Tyr Tyr Ser Asp Ser Val Lys Gly Arg Phe Thr
        195                 200                 205

Ile Ser Arg Asp Asp Ser Lys Ser Ser Val Tyr Leu Gln Met Asn Asn
    210                 215                 220

Leu Arg Val Glu Asp Thr Gly Ile Tyr Tyr Cys Thr Gly Ala Ser Tyr
225                 230                 235                 240

Gly Met Glu Tyr Leu Gly Gln Gly Thr Ser Val Thr Val Ser
                245                 250

<210>  45
<211>  15
<212>  PRT
<213>  artificial sequence

<220>
<223>  antibody sequence

<400>  45

Gly Gly Gly Gly Ser Gly Gly Gly Gly Ser Gly Gly Gly Gly Ser
1               5                   10                  15

<210>  46
<211>  24
<212>  PRT
<213>  artificial sequence

<220>
<223>  antibody sequence

<400>  46

Ala Asp Asp Ala Lys Lys Asp Ala Ala Lys Lys Asp Asp Ala Lys Lys
1               5                   10                  15

Asp Asp Ala Lys Lys Asp Gly Gly
            20

<210>  47
<211>  184
<212>  PRT
<213>  artificial sequence

<220>
<223>  antibody sequence

<400>  47

Asp Val Tyr His Asp Gly Ala Cys Pro Glu Val Lys Pro Val Asp Asn
1               5                   10                  15

Phe Asp Trp Ser Gln Tyr His Gly Lys Trp Trp Glu Val Ala Lys Tyr
            20                  25                  30

Pro Ser Pro Asn Gly Lys Tyr Gly Lys Cys Gly Trp Ile Glu Tyr Thr
        35                  40                  45

Pro Glu Gly Lys Ser Val Lys Val Ser Arg Tyr Asp Val Ile His Gly
    50                  55                  60

Lys Glu Tyr Phe Met Glu Gly Thr Ala Tyr Pro Val Gly Asp Ser Lys
65                  70                  75                  80

Ile Gly Lys Ile Tyr His Ser Arg Thr Val Gly Gly Tyr Thr Lys Lys
                85                  90                  95

Thr Val Phe Asn Val Leu Ser Thr Asp Asn Lys Asn Tyr Ile Ile Gly
            100                 105                 110

Tyr Arg Cys Arg Tyr Asp Glu Asp Lys Lys Gly His Trp Asp His Val
        115                 120                 125

Trp Val Leu Ser Arg Ser Met Val Leu Thr Gly Glu Ala Lys Thr Ala
    130                 135                 140

Val Glu Asn Tyr Leu Ile Gly Ser Pro Val Val Asp Ser Gln Lys Leu
145                 150                 155                 160

Val Tyr Ser Asp Phe Ser Glu Ala Ala Cys Lys Val Asn Asn Ser Asn
                165                 170                 175

Trp Ser His Pro Gln Phe Glu Lys
            180

<210>  48
<211>  505
<212>  PRT
<213>  artificial sequence

<220>
<223>  antibody sequence

<400>  48

Gln Val Gln Leu Val Glu Ser Gly Gly Asn Leu Val Gln Pro Gly Gly
1               5                   10                  15

Ser Leu Arg Leu Ser Cys Ala Ala Ser Gly Phe Thr Phe Gly Ser Phe

20                  25                  30

Ser Met Ser Trp Val Arg Gln Ala Pro Gly Gly Gly Leu Glu Trp Val
        35                  40                  45

Ala Gly Leu Ser Ala Arg Ser Ser Leu Thr His Tyr Ala Asp Ser Val
    50                  55                  60

Lys Gly Arg Phe Thr Ile Ser Arg Asp Asn Ala Lys Asn Ser Val Tyr
65                  70                  75                  80

Leu Gln Met Asn Ser Leu Arg Val Glu Asp Thr Ala Val Tyr Tyr Cys
                85                  90                  95

Ala Arg Arg Ser Tyr Asp Ser Ser Gly Tyr Trp Gly His Phe Tyr Ser
            100                 105                 110

Tyr Met Asp Val Trp Gly Gln Gly Thr Leu Val Thr Val Ser Gly Gly
        115                 120                 125

Gly Gly Ser Gly Gly Gly Gly Ser Gly Gly Gly Gly Ser Gln Ser Val
    130                 135                 140

Leu Thr Gln Pro Ser Ser Val Ser Ala Ala Pro Gly Gln Lys Val Thr
145                 150                 155                 160

Ile Ser Cys Ser Gly Ser Thr Ser Asn Ile Gly Asn Asn Tyr Val Ser
                165                 170                 175

Trp Tyr Gln Gln His Pro Gly Lys Ala Pro Lys Leu Met Ile Tyr Asp
            180                 185                 190

Val Ser Lys Arg Pro Ser Gly Val Pro Asp Arg Phe Ser Gly Ser Lys
        195                 200                 205

Ser Gly Asn Ser Ala Ser Leu Asp Ile Ser Gly Leu Gln Ser Glu Asp
    210                 215                 220

Glu Ala Asp Tyr Tyr Cys Ala Ala Trp Asp Asp Ser Leu Ser Glu Phe
225                 230                 235                 240

Leu Phe Gly Thr Gly Thr Lys Leu Thr Val Leu Gly Gly Gly Gly Ser
                245                 250                 255

Asp Ile Lys Leu Gln Gln Ser Gly Ala Glu Leu Ala Arg Pro Gly Ala
            260                 265                 270

Ser Val Lys Met Ser Cys Lys Thr Ser Gly Tyr Thr Phe Thr Arg Tyr
        275                 280                 285

Thr Met His Trp Val Lys Gln Arg Pro Gly Gln Gly Leu Glu Trp Ile
    290                 295                 300

Gly Tyr Ile Asn Pro Ser Arg Gly Tyr Thr Asn Tyr Asn Gln Lys Phe
305                 310                 315                 320

Lys Asp Lys Ala Thr Leu Thr Thr Asp Lys Ser Ser Ser Thr Ala Tyr
                325                 330                 335

Met Gln Leu Ser Ser Leu Thr Ser Glu Asp Ser Ala Val Tyr Tyr Cys
            340                 345                 350

Ala Arg Tyr Tyr Asp Asp His Tyr Cys Leu Asp Tyr Trp Gly Gln Gly
        355                 360                 365

Thr Thr Leu Thr Val Ser Ser Val Glu Gly Gly Ser Gly Gly Ser Gly
    370                 375                 380

Gly Ser Gly Gly Ser Gly Gly Val Asp Asp Ile Gln Leu Thr Gln Ser
385                 390                 395                 400

Pro Ala Ile Met Ser Ala Ser Pro Gly Glu Lys Val Thr Met Thr Cys
                405                 410                 415

Arg Ala Ser Ser Ser Val Ser Tyr Met Asn Trp Tyr Gln Gln Lys Ser
            420                 425                 430

Gly Thr Ser Pro Lys Arg Trp Ile Tyr Asp Thr Ser Lys Val Ala Ser
        435                 440                 445

Gly Val Pro Tyr Arg Phe Ser Gly Ser Gly Ser Gly Thr Ser Tyr Ser
    450                 455                 460

Leu Thr Ile Ser Ser Met Glu Ala Glu Asp Ala Ala Thr Tyr Tyr Cys
465                 470                 475                 480

Gln Gln Trp Ser Ser Asn Pro Leu Thr Phe Gly Ala Gly Thr Lys Leu
                485                 490                 495

Glu Leu Lys His His His His His His
            500                 505

<210>  49
<211>  508
<212>  PRT

<213>  artificial sequence

<220>
<223>  antibody sequence

<400>  49

Asp Val Val Met Thr Gln Thr Pro Leu Ser Leu Pro Val Ser Leu Gly
1               5                   10                  15

Asp Gln Ala Ser Ile Ser Cys Arg Ser Ser Gln Ser Leu Val His Ser
            20                  25                  30

Asn Gly Asn Thr Tyr Leu Arg Trp Tyr Leu Gln Lys Pro Gly Gln Ser
        35                  40                  45

Pro Lys Val Leu Ile Tyr Lys Val Ser Asn Arg Val Ser Gly Val Pro
    50                  55                  60

Asp Arg Phe Ser Gly Ser Gly Ser Gly Thr Asp Phe Thr Leu Lys Ile
65                  70                  75                  80

Asn Arg Val Glu Ala Glu Asp Leu Gly Val Tyr Phe Cys Ser Gln Ser
                85                  90                  95

Thr His Val Pro Trp Thr Phe Gly Gly Gly Thr Lys Leu Glu Ile Lys
            100                 105                 110

Ser Ser Ala Asp Asp Ala Lys Lys Asp Ala Ala Lys Lys Asp Asp Ala
        115                 120                 125

Lys Lys Asp Asp Ala Lys Lys Asp Gly Gly Val Lys Leu Asp Glu Thr
    130                 135                 140

Gly Gly Gly Leu Val Gln Pro Gly Gly Ala Met Lys Leu Ser Cys Val
145                 150                 155                 160

Thr Ser Gly Phe Thr Phe Gly His Tyr Trp Met Asn Trp Val Arg Gln
                165                 170                 175

Ser Pro Glu Lys Gly Leu Glu Trp Val Ala Gln Phe Arg Asn Lys Pro
            180                 185                 190

Tyr Asn Tyr Glu Thr Tyr Tyr Ser Asp Ser Val Lys Gly Arg Phe Thr
        195                 200                 205

Ile Ser Arg Asp Asp Ser Lys Ser Ser Val Tyr Leu Gln Met Asn Asn
    210                 215                 220

Leu Arg Val Glu Asp Thr Gly Ile Tyr Tyr Cys Thr Gly Ala Ser Tyr
225                 230                 235                 240

Gly Met Glu Tyr Leu Gly Gln Gly Thr Ser Val Thr Val Ser Gly Gly
                245                 250                 255

Gly Gly Ser Asp Ile Lys Leu Gln Gln Ser Gly Ala Glu Leu Ala Arg
            260                 265                 270

Pro Gly Ala Ser Val Lys Met Ser Cys Lys Thr Ser Gly Tyr Thr Phe
        275                 280                 285

Thr Arg Tyr Thr Met His Trp Val Lys Gln Arg Pro Gly Gln Gly Leu
    290                 295                 300

Glu Trp Ile Gly Tyr Ile Asn Pro Ser Arg Gly Tyr Thr Asn Tyr Asn
305                 310                 315                 320

Gln Lys Phe Lys Asp Lys Ala Thr Leu Thr Thr Asp Lys Ser Ser Ser
                325                 330                 335

Thr Ala Tyr Met Gln Leu Ser Ser Leu Thr Ser Glu Asp Ser Ala Val
            340                 345                 350

Tyr Tyr Cys Ala Arg Tyr Tyr Asp Asp His Tyr Cys Leu Asp Tyr Trp
        355                 360                 365

Gly Gln Gly Thr Thr Leu Thr Val Ser Ser Val Glu Gly Gly Ser Gly
    370                 375                 380

Gly Ser Gly Gly Ser Gly Gly Ser Gly Gly Val Asp Asp Ile Gln Leu
385                 390                 395                 400

Thr Gln Ser Pro Ala Ile Met Ser Ala Ser Pro Gly Glu Lys Val Thr
                405                 410                 415

Met Thr Cys Arg Ala Ser Ser Ser Val Ser Tyr Met Asn Trp Tyr Gln
            420                 425                 430

Gln Lys Ser Gly Thr Ser Pro Lys Arg Trp Ile Tyr Asp Thr Ser Lys
        435                 440                 445

Val Ala Ser Gly Val Pro Tyr Arg Phe Ser Gly Ser Gly Ser Gly Thr
    450                 455                 460

Ser Tyr Ser Leu Thr Ile Ser Ser Met Glu Ala Glu Asp Ala Ala Thr
465                 470                 475                 480

Tyr Tyr Cys Gln Gln Trp Ser Ser Asn Pro Leu Thr Phe Gly Ala Gly
                485                 490                 495

Thr Lys Leu Glu Leu Lys His His His His His His
            500                 505

<210> 50
<211> 438
<212> PRT
<213> artificial sequence

<220>
<223> antibody sequence

<400> 50

Asp Val Tyr His Asp Gly Ala Cys Pro Glu Val Lys Pro Val Asp Asn
1               5                   10                  15

Phe Asp Trp Ser Gln Tyr His Gly Lys Trp Trp Glu Val Ala Lys Tyr
            20                  25                  30

Pro Ser Pro Asn Gly Lys Tyr Gly Lys Cys Gly Trp Ile Glu Tyr Thr
        35                  40                  45

Pro Glu Gly Lys Ser Val Lys Val Ser Arg Tyr Asp Val Ile His Gly
    50                  55                  60

Lys Glu Tyr Phe Met Glu Gly Thr Ala Tyr Pro Val Gly Asp Ser Lys
65                  70                  75                  80

Ile Gly Lys Ile Tyr His Ser Arg Thr Val Gly Gly Tyr Thr Lys Lys
                85                  90                  95

Thr Val Phe Asn Val Leu Ser Thr Asp Asn Lys Asn Tyr Ile Ile Gly
            100                 105                 110

Tyr Arg Cys Arg Tyr Asp Glu Asp Lys Lys Gly His Trp Asp His Val
        115                 120                 125

Trp Val Leu Ser Arg Ser Met Val Leu Thr Gly Glu Ala Lys Thr Ala
    130                 135                 140

Val Glu Asn Tyr Leu Ile Gly Ser Pro Val Val Asp Ser Gln Lys Leu
145                 150                 155                 160

Val Tyr Ser Asp Phe Ser Glu Ala Ala Cys Lys Val Asn Asn Ser Asn
                165                 170                 175

Trp Ser His Pro Gln Phe Glu Lys Gly Gly Gly Gly Ser Asp Ile Lys
        180                 185                 190

Leu Gln Gln Ser Gly Ala Glu Leu Ala Arg Pro Gly Ala Ser Val Lys
    195                 200                 205

Met Ser Cys Lys Thr Ser Gly Tyr Thr Phe Thr Arg Tyr Thr Met His
    210                 215                 220

Trp Val Lys Gln Arg Pro Gly Gln Gly Leu Glu Trp Ile Gly Tyr Ile
225                 230                 235                 240

Asn Pro Ser Arg Gly Tyr Thr Asn Tyr Asn Gln Lys Phe Lys Asp Lys
                245                 250                 255

Ala Thr Leu Thr Thr Asp Lys Ser Ser Ser Thr Ala Tyr Met Gln Leu
            260                 265                 270

Ser Ser Leu Thr Ser Glu Asp Ser Ala Val Tyr Tyr Cys Ala Arg Tyr
        275                 280                 285

Tyr Asp Asp His Tyr Cys Leu Asp Tyr Trp Gly Gln Gly Thr Thr Leu
    290                 295                 300

Thr Val Ser Ser Val Glu Gly Gly Ser Gly Gly Ser Gly Gly Ser Gly
305                 310                 315                 320

Gly Ser Gly Gly Val Asp Asp Ile Gln Leu Thr Gln Ser Pro Ala Ile
                325                 330                 335

Met Ser Ala Ser Pro Gly Glu Lys Val Thr Met Thr Cys Arg Ala Ser
            340                 345                 350

Ser Ser Val Ser Tyr Met Asn Trp Tyr Gln Gln Lys Ser Gly Thr Ser
        355                 360                 365

Pro Lys Arg Trp Ile Tyr Asp Thr Ser Lys Val Ala Ser Gly Val Pro
    370                 375                 380

Tyr Arg Phe Ser Gly Ser Gly Ser Gly Thr Ser Tyr Ser Leu Thr Ile
385                 390                 395                 400

Ser Ser Met Glu Ala Glu Asp Ala Ala Thr Tyr Tyr Cys Gln Gln Trp
                405                 410                 415

Ser Ser Asn Pro Leu Thr Phe Gly Ala Gly Thr Lys Leu Glu Leu Lys
            420                 425                 430

His His His His His His
        435

<210>  51
<211>  265
<212>  PRT
<213>  artificial sequence

<220>
<223>  antibody sequence

<400>  51

Asp Ile Lys Leu Gln Gln Ser Gly Ala Glu Leu Ala Arg Pro Gly Ala
1               5                   10                  15

Ser Val Lys Met Ser Cys Lys Thr Ser Gly Tyr Thr Phe Thr Arg Tyr
            20                  25                  30

Thr Met His Trp Val Lys Gln Arg Pro Gly Gln Gly Leu Glu Trp Ile
        35                  40                  45

Gly Tyr Ile Asn Pro Ser Arg Gly Tyr Thr Asn Tyr Asn Gln Lys Phe
    50                  55                  60

Lys Asp Lys Ala Thr Leu Thr Thr Asp Lys Ser Ser Ser Thr Ala Tyr
65                  70                  75                  80

Met Gln Leu Ser Ser Leu Thr Ser Glu Asp Ser Ala Val Tyr Tyr Cys
                85                  90                  95

Ala Arg Tyr Tyr Asp Asp His Tyr Cys Leu Asp Tyr Trp Gly Gln Gly
            100                 105                 110

Thr Thr Leu Thr Val Ser Ser Val Glu Gly Gly Ser Gly Gly Ser Gly
        115                 120                 125

Gly Ser Gly Gly Ser Gly Gly Val Asp Asp Ile Gln Leu Thr Gln Ser
    130                 135                 140

Pro Ala Ile Met Ser Ala Ser Pro Gly Glu Lys Val Thr Met Thr Cys
145                 150                 155                 160

Arg Ala Ser Ser Ser Val Ser Tyr Met Asn Trp Tyr Gln Gln Lys Ser
                165                 170                 175

Gly Thr Ser Pro Lys Arg Trp Ile Tyr Asp Thr Ser Lys Val Ala Ser
            180                 185                 190

Gly Val Pro Tyr Arg Phe Ser Gly Ser Gly Ser Gly Thr Ser Tyr Ser
        195                 200                 205

Leu Thr Ile Ser Ser Met Glu Ala Glu Asp Ala Ala Thr Tyr Tyr Cys
    210                 215                 220

Gln Gln Trp Ser Ser Asn Pro Leu Thr Phe Gly Ala Gly Thr Lys Leu
225                 230                 235                 240

Glu Leu Lys Ala Ala Ala Glu Gln Lys Leu Ile Ser Glu Glu Asp Leu
                245                 250                 255

Gly Ser His His His His His His Cys
            260                 265

<210>  52
<211>  118
<212>  PRT
<213>  artificial sequence

<220>
<223>  antibody sequence

<400>  52

Ser Glu Val Gln Leu Val Glu Ser Gly Gly Gly Leu Val Gln Pro Gly
1               5                   10                  15

Gly Ser Leu Arg Leu Ser Cys Ala Ala Ser Gly Phe Asn Ile Ser Val
            20                  25                  30

Tyr Met Met His Trp Val Arg Gln Ala Pro Gly Lys Gly Leu Glu Trp
        35                  40                  45

Val Ala Ser Ile Tyr Pro Tyr Ser Gly Tyr Thr Tyr Tyr Ala Asp Ser
    50                  55                  60

Val Lys Gly Arg Phe Thr Ile Ser Ala Asp Thr Ser Lys Asn Thr Ala
65                  70                  75                  80

Tyr Leu Gln Met Asn Ser Leu Arg Ala Glu Asp Thr Ala Val Tyr Tyr
                85                  90                  95

Cys Ala Arg Tyr Val Tyr His Ala Leu Asp Tyr Trp Gly Gln Gly Thr
            100                 105                 110

Leu Val Thr Val Ser Ser
        115

<210>  53

<211>  9
<212>  PRT
<213>  artificial sequence

<220>
<223>  antibody sequence

<400>  53

Phe Asn Ile Ser Val Tyr Met Met His
1               5


<210>  54
<211>  14
<212>  PRT
<213>  artificial sequence

<220>
<223>  antibody sequence

<400>  54

Trp Val Ala Ser Ile Tyr Pro Tyr Ser Gly Tyr Thr Tyr Tyr
1               5                   10


<210>  55
<211>  9
<212>  PRT
<213>  artificial sequence

<220>
<223>  antibody sequence

<400>  55

Arg Tyr Val Tyr His Ala Leu Asp Tyr
1               5


<210>  56
<211>  7
<212>  PRT
<213>  artificial sequence

<220>
<223>  antibody sequence

<400>  56

Gly Phe Asn Ile Ser Val Tyr
1               5


<210>  57
<211>  6
<212>  PRT
<213>  artificial sequence

<220>
<223>  antibody sequence

<400>  57

Tyr Pro Tyr Ser Gly Tyr
1               5


<210>  58
<211>  8
<212>  PRT
<213>  artificial sequence

<220>
<223>  antibody sequence

<400>  58

Tyr Val Tyr His Ala Leu Asp Tyr
1               5


<210>  59
<211>  111
<212>  PRT
<213>  artificial sequence

<220>
<223>  antibody sequence

<400>  59

Asp Ile Gln Met Thr Gln Ser Pro Ser Ser Leu Ser Ala Ser Val Gly
1               5                   10                  15

Asp Arg Val Thr Ile Thr Cys Arg Ala Ser Gln Arg Gly Leu Arg Asn
            20                  25                  30

Val Ala Val Ala Trp Tyr Gln Gln Lys Pro Gly Lys Ala Pro Lys Leu
        35                  40                  45

Leu Ile Tyr Ser Ala Ser Ser Leu Tyr Ser Gly Val Pro Ser Arg Phe
    50                  55                  60

Ser Gly Ser Arg Ser Gly Thr Asp Phe Thr Leu Thr Ile Ser Ser Leu
65                  70                  75                  80

Gln Pro Glu Asp Phe Ala Thr Tyr Tyr Cys Gln Gln Trp Ala Val His
                85                  90                  95

Ser Leu Ile Thr Phe Gly Gln Gly Thr Lys Val Glu Ile Lys Arg
            100                 105                 110


<210>  60
<211>  10
<212>  PRT
<213>  artificial sequence

<220>
<223>  antibody sequence

<400>  60

Gln Arg Gly Leu Arg Asn Val Ala Val Ala
1               5                   10


<210>  61
<211>  11
<212>  PRT
<213>  artificial sequence

<220>
<223>  antibody sequence

<400>  61

Leu Leu Ile Tyr Ser Ala Ser Ser Leu Tyr Ser
1               5                   10


<210>  62
<211>  9
<212>  PRT
<213>  artificial sequence

<220>
<223>  antibody sequence

<400>  62

Gln Gln Trp Ala Val His Ser Leu Ile
1               5


<210>  63
<211>  13
<212>  PRT
<213>  artificial sequence

<220>
<223>  antibody sequence

<400>  63

Arg Ala Ser Gln Arg Gly Leu Arg Asn Val Ala Val Ala
1               5                   10


<210>  64
<211>  7
<212>  PRT
<213>  artificial sequence

<220>
<223>  antibody sequence

<400>  64

Ser Ala Ser Ser Leu Tyr Ser
1               5

<210>  65
<211>  10
<212>  PRT
<213>  artificial sequence

<220>
<223>  antibody sequence

<400>  65

Gln Gln Trp Ala Val His Ser Leu Ile Thr
1               5                   10


<210>  66
<211>  243
<212>  PRT
<213>  artificial sequence

<220>
<223>  antibody sequence

<400>  66

Asp Ile Gln Met Thr Gln Ser Pro Ser Ser Leu Ser Ala Ser Val Gly
1               5                   10                  15

Asp Arg Val Thr Ile Thr Cys Arg Ala Ser Gln Arg Gly Leu Arg Asn
            20                  25                  30

Val Ala Val Ala Trp Tyr Gln Gln Lys Pro Gly Lys Ala Pro Lys Leu
        35                  40                  45

Leu Ile Tyr Ser Ala Ser Ser Leu Tyr Ser Gly Val Pro Ser Arg Phe
    50                  55                  60

Ser Gly Ser Arg Ser Gly Thr Asp Phe Thr Leu Thr Ile Ser Ser Leu
65                  70                  75                  80

Gln Pro Glu Asp Phe Ala Thr Tyr Tyr Cys Gln Gln Trp Ala Val His
                85                  90                  95

Ser Leu Ile Thr Phe Gly Gln Gly Thr Lys Val Glu Ile Lys Arg Gly
            100                 105                 110

Gly Gly Gly Ser Gly Gly Gly Gly Ser Gly Gly Gly Gly Ser Glu Val
        115                 120                 125

Gln Leu Val Glu Ser Gly Gly Gly Leu Val Gln Pro Gly Gly Ser Leu
    130                 135                 140

Arg Leu Ser Cys Ala Ala Ser Gly Phe Asn Ile Ser Val Tyr Met Met

145                 150                 155                 160

His Trp Val Arg Gln Ala Pro Gly Lys Gly Leu Glu Trp Val Ala Ser
                165                 170                 175

Ile Tyr Pro Tyr Ser Gly Tyr Thr Tyr Tyr Ala Asp Ser Val Lys Gly
            180                 185                 190

Arg Phe Thr Ile Ser Ala Asp Thr Ser Lys Asn Thr Ala Tyr Leu Gln
        195                 200                 205

Met Asn Ser Leu Arg Ala Glu Asp Thr Ala Val Tyr Tyr Cys Ala Arg
    210                 215                 220

Tyr Val Tyr His Ala Leu Asp Tyr Trp Gly Gln Gly Thr Leu Val Thr
225                 230                 235                 240

Val Ser Ser

<210>  67
<211>  5
<212>  PRT
<213>  artificial sequence

<220>
<223>  antibody sequence

<400>  67

Gly Gly Gly Gly Ser
1               5

<210>  68
<211>  116
<212>  PRT
<213>  artificial sequence

<220>
<223>  antibody sequence

<400>  68

Glu Val Gln Leu Leu Glu Ser Gly Gly Gly Leu Val Gln Pro Gly Gly
1               5                   10                  15

Ser Leu Arg Leu Ser Cys Ala Ala Ser Gly Phe Thr Phe Ser Ser Tyr
            20                  25                  30

Ala Met Ser Trp Val Arg Gln Ala Pro Gly Lys Gly Leu Glu Trp Val
        35                  40                  45

Ser Ala Ile Asp Gly Ser Gly Gly Ser Thr Tyr Tyr Ala Asp Ser Val
    50                  55                  60

Lys Gly Arg Phe Thr Ile Ser Arg Asp Asn Ser Lys Asn Thr Leu Tyr
65                  70                  75                  80

Leu Gln Met Asn Ser Leu Arg Ala Glu Asp Thr Ala Val Tyr Tyr Cys
                85                  90                  95

Val Lys Gly Pro Pro Val Phe Asp Tyr Trp Gly Gln Gly Thr Leu Val
            100                 105                 110

Thr Val Ser Ser
        115

<210>  69
<211>  9
<212>  PRT
<213>  artificial sequence

<220>
<223>  antibody sequence

<400>  69

Phe Thr Phe Ser Ser Tyr Ala Met Ser
1               5

<210>  70
<211>  15
<212>  PRT
<213>  artificial sequence

<220>
<223>  antibody sequence

<400>  70

Trp Val Ser Ala Ile Asp Gly Ser Gly Gly Ser Thr Tyr Tyr Ala
1               5                   10                  15

<210>  71
<211>  9
<212>  PRT
<213>  artificial sequence

<220>
<223>  antibody sequence

<400>  71

Val Lys Gly Pro Pro Val Phe Asp Tyr
1               5

<210>  72

<211>  108
<212>  PRT
<213>  artificial sequence

<220>
<223>  antibody sequence

<400>  72

Ser Ser Glu Leu Thr Gln Asp Pro Ala Val Ser Val Ala Leu Gly Gln
1               5                   10                  15

Thr Val Arg Ile Thr Cys Gln Gly Asp Ser Leu Arg Ser Tyr Tyr Ala
            20                  25                  30

Ser Trp Tyr Gln Gln Lys Pro Gly Gln Ala Pro Val Leu Val Ile Tyr
        35                  40                  45

Gly Lys Asn Asn Arg Pro Ser Gly Ile Pro Asp Arg Phe Ser Gly Ser
    50                  55                  60

Ser Ser Gly Asn Thr Ala Ser Leu Thr Ile Thr Gly Ala Gln Ala Glu
65                  70                  75                  80

Asp Glu Ala Asp Tyr Tyr Cys Gln Ser Ser Lys Trp Ser Trp Asp Pro
                85                  90                  95

Val Val Phe Gly Gly Gly Thr Lys Leu Thr Val Leu
            100                 105


<210>  73
<211>  8
<212>  PRT
<213>  artificial sequence

<220>
<223>  antibody sequence

<400>  73

Ser Leu Arg Ser Tyr Tyr Ala Ser
1               5


<210>  74
<211>  11
<212>  PRT
<213>  artificial sequence

<220>
<223>  antibody sequence

<400>  74

Leu Val Ile Tyr Gly Lys Asn Asn Arg Pro Ser
1               5                   10

<210>  75
<211>  10
<212>  PRT
<213>  artificial sequence

<220>
<223>  antibody sequence

<400>  75

Gln Ser Ser Lys Trp Ser Trp Asp Pro Val
1               5                   10